(19) Europäisches Patentamt
European Patent Office
Office européen des brevets

(11) EP 3 044 163 B1

(12) **EUROPEAN PATENT SPECIFICATION**

(45) Date of publication and mention of the grant of the patent:
**07.07.2021 Bulletin 2021/27**

(21) Application number: **14766435.3**

(22) Date of filing: **09.09.2014**

(51) Int Cl.:
***B82Y 30/00*** *(2011.01)* ***A61Q 3/02*** *(2006.01)*
***A61K 8/25*** *(2006.01)* ***A61K 9/14*** *(2006.01)*
***A61L 24/00*** *(2006.01)* ***A61L 24/02*** *(2006.01)*
***A61L 27/10*** *(2006.01)* ***A61L 27/12*** *(2006.01)*
***A61L 27/50*** *(2006.01)* ***A61L 27/52*** *(2006.01)*

(86) International application number:
**PCT/EP2014/069233**

(87) International publication number:
**WO 2015/036410 (19.03.2015 Gazette 2015/11)**

(54) **NANOPARTICLES FOR USE IN BIOADHESION**
NANOPARTIKEL ZUR VERWENDUNG IN BIOADHÄSION
NANOPARTICULES POUR UTILISATION EN BIOADHÉRENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.09.2013 EP 13306243**

(43) Date of publication of application:
**20.07.2016 Bulletin 2016/29**

(73) Proprietor: **ESPCI PARISTECH**
**75005 Paris (FR)**

(72) Inventors:
- **MARCELLAN, Alba**
  **F-94600 Choisy-le-Roi (FR)**
- **LEIBLER, Ludwik**
  **F-75006 Paris (FR)**

(74) Representative: **Corizzi, Valérie**
**PACT-IP**
**37, rue Royale**
**92210 Saint-Cloud (FR)**

(56) References cited:
**WO-A2-02/41765** **US-A1- 2009 275 526**
**US-A1- 2013 052 712**

- **MAHESHWARI M ET AL: "DEVELOPMENT OF TETRACYCLINE-SERRATIOPEPTIDASE-CONTAINING PERIODONTAL GEL: FORMULATION AND PRELIMINARY CLINICAL STUDY", AAPS PHARMSCITECH, SPRINGER NEW YORK LLC, US, vol. 7, no. 3, 1 January 2006 (2006-01-01) , pages E1-E10, XP002547740, ISSN: 1530-9932, DOI: 10.1208/PT070376 [retrieved on 2006-09-15]**
- **AGUZZI ET AL: "Use of clays as drug delivery systems: Possibilities and limitations", APPLIED CLAY SCIENCE, ELSEVIER SCIENCE, NL, vol. 36, no. 1-3, 16 March 2007 (2007-03-16), pages 22-36, XP005932574, ISSN: 0169-1317, DOI: 10.1016/J.CLAY.2006.06.015**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 044 163 B1

## Description

### FIELD OF THE INVENTION

The invention relates to the use of inorganic nanoparticles, notably aqueous suspensions of nanoparticles, as a gluing agent between a hydrogel, which can be a synthetic gel or a biological tissue, and a second article, of a material which can be identical to or different from the hydrogel. It gives access to assemblies of at least one hydrogel and at least a second article, said assemblies comprising an interface of nanoparticles. The invention gives access to gluing kits based on nanoparticles, notably on nanoparticles suspensions. Using a composition of inorganic nanoparticles, notably a dispersion or suspension of nanoparticles for gluing a gel to another article finds applications in numerous technical fields, like microfluidics, laboratory equipment, actuation, surgery, tissue engineering, drug delivery, agrochemical industry.

### BACKGROUND OF THE INVENTION

Synthetic gels find numerous applications, however, when using hydrogels, one difficulty lies in creating a bonding between a gel and another article, of an identical or different material. First, bonding efficiently a gel to another article in itself is difficult, then creating a bond, which is resistant to the environmental conditions of use of the assembly (e.g. immersion in water) is another difficulty. Hydrogels are often fragile materials. Making a more resistant assembly with a more resistant gel or material could be an envisaged solution. Unfortunately, such assemblies are often characterized by a weak bonding of the gel to other parts of the assembly. Using adhesives could alleviate these difficulties. Ideally adhesives should bring adequate bonding without compromising osmotic and mechanical responsiveness of gels.

Adhesives are generally made of polymers (Kendall, K. Molecular Adhesion and Its Applications, Plenum Publishing Corporation, 2001). Polymers, in contrast to other materials, are able to ensure good contact by filling surface asperities and retard fracture of adhesive joint by dissipating energy under stress (Lake, G. J. & Thomas, A. G. Proceedings of the Royal Society A: Mathematical, Physical and Engineering Sciences 300, 108-119 (1967); De Gennes, P. G., Langmuir 12, 4497-4500 (1996)). Paradoxically, using polymers to assemble polymer gels is challenging and forming an adhesive junction requires chemical reactions, heating, changing pH, using UV irradiation or applying electric field (Sahlin, J. J. & Peppas, N. A., Journal of Biomaterials Science, Polymer Edition 8, 421-436 (1997); Tamagawa, H. & Takahashi, Y., Materials Chemistry and Physics 107, 164-170 (2008) ; Saito, J. et al., Polymer Chemistry 2, 575 (2011); Techawanitchai, P. et $\alpha$/., Soft Matter 8, 2844 (2012)).

Most often, when brought into contact and pressed together, two pieces of an elastic gel do not stick and gel-gel friction is very low (Gong, J. P., Soft Matter 2, 544 (2006)). Incorporating supramolecular or covalent reversible bonds in polymers enables one to produce gels that are self-adhesive (Pezron, E., Ricard, A. & Leibler, L., J. Polym. Sci. B Polym. Phys. 28, 2445-2461 (1990) ; Bosman, A. W., Sijbesma, R. P. & Meijer, E. W., Materials Today, 34-39 (2004) ; Reutenauer, P., Buhler, E., Boul, P. J., Candau, S. J. & Lehn, J.-M., Chemistry 15, 1893-1900 (2009) ; Nicolay, R., Kamada, J., Van Wassen, A. & Matyjaszewski, K., Macromolecules 43, 4355-4361 (2010); Imato, K. et al., Angew. Chem. Int. Ed. 51, 1138-1142 (2011)). However, manipulating self-adhesive gels is not always practical. A solution could be to use supramolecular networks with surfaces that are not self-adhesive, but which are able to self-heal when cut into pieces (Cordier, P., Tournilhac, F., Soulié-Ziakovic, C. & Leibler, L., Nature 451, 977-980 (2008) ; Maes, F. et al., Soft Matter 8, 1681-1687 (2012)). Nanocomposites comprising a hydrogel wherein nanoparticles are embedded have been disclosed by different authors. Pressure sensitive adhesives based on hydrogel nanocomposites have been suggested for skin contact applications (Bait N. et al., Soft Matter 7, 2025-2032 (2011); Aguzzi et al., Applied Clay Science, vol.36, n°1-3, p.22-36 (2007)). Notably, dispersing clay particles in polymer solutions or networks yields gels that combine adhesive properties (and also high elasticity and toughness) with self-healing capabilities (Wang, Q. et al., Nature 463, 339-343 (2010); Haraguchi, K., Uyama, K. & Tanimoto, H. Macromol. Rapid Commun. 32, 1253-1258 (2011)). In this design clay particles play a role of reversible cross-links (Carlsson, L., Rose, S., Hourdet, D. & Marcellan, A., Soft Matter 6, 3619-3631 (2010); Gaharwar, A. K., Rivera, C. P., Wu, C.-J. & Schmidt, G., Acta Biomaterialia 7, 4139-4148 (2011)). Still, any particular gel cannot answer all demands and it remains desirable to find a practical and universal means to glue together or increase adhesion between any biological or synthetic gel or biological tissues.

Further, these prior art solutions for gel adhesion are not deprived of inconvenient. Particularly, it has been noted that the dispersion of nanoparticles in a gel material modifies the intrinsic properties of the gel, notably, it increases its stiffness. For biological tissues this solution is not practical at all.

To the difference of the prior art, the adhesive is made of nanoparticle solution or nanoparticle powder and the nanoparticles, preferably solid, are applied and located at the interface between the two adherents, i.e. the hydrogel and the other article, instead of being included in the hydrogel formulation.

The invention permits to glue or to create adhesion or to increase adhesion of a hydrogel to another article. The invention finds application in creating adhesion of a hydrogel to itself when the hydrogel is not self-adhesive, for

example when the hydrogel is not a hydrogel nanocomposite. The invention permits increasing adhesion of a hydrogel to another article or to itself, in cases wherein the hydrogel is only weakly self-adhesive. The invention differs from using nanocomposite hydrogels as an adhesive notably in that the nanoparticles composition is distinct from the composition of the hydrogel and from the other article.

Nanoparticles have been used in the medical field for many applications and are therefore generally considered as safe for application to humans.

WO2004/045494 discloses thrombin-conjugated nanoparticles for the preparation of fibrin-based biological glue. The formulation permits rapid formation of the fibrin clot. The fibrin glue itself acts as gluing agent. WO2004/045494 does not disclose the use of nanoparticles which are not conjugated to thrombin in an adhesion or gluing method.

WO2006/012541 discloses wound sealants based upon a binding agent of reactive submicron silica particles that, when hydrated, agglomerate in the form of a supramolecular cross-linked network which facilitates clot formation. The clot forms a dressing on the tissue. The dressing is not an adhesive or glue. It is a layer which closes and protects the wound. Documents WO2004/045494 and WO2006/012541 do not disclose the use of nanoparticles as an adhesive between at least one hydrogel and at least one other article. These documents do not disclose the adhesion promoting properties of nanoparticles when applied to a gel of biological or synthetic nature. These documents do not disclose the use of nanoparticles for gluing or creating adhesion between at least one hydrogel and at least one other article, wherein the article and the hydrogel are not biological tissues. These documents do not disclose the use of nanoparticles for gluing or creating adhesion between at least one hydrogel and at least one other article in the absence of clot formation. WO03/026481 discloses a method for tissue repair, which uses magnetic nanoparticles. Nanoparticles are delivered to the site to be repaired and submitted to an excitation source under conditions wherein they emit heat. This method also permits joining non-tissue materials, for example polymers. However, the nanoparticles have the function of heating agent, in a method wherein heating promotes welding of the tissues. This document does not disclose the use of nanoparticles as gluing agent between a hydrogel and another article. This document does not disclose the use of nanoparticles for gluing a hydrogel and another article without exposure to laser radiation. This document does not disclose the use of nanoparticles for gluing a hydrogel and another article without heating the nanoparticles. Notably, according to the invention nanoparticles provide adhesion at ambient or body temperature.

The invention differs from this prior art notably in that the nanoparticles provide adhesion without heating. For this reason, they are much more easy to use. They also permit adhesion between a hydrogel and another article, none being a biological tissue.

US2009275526 relates to a pharmaceutical composition which includes mucoadhesive nanoparticles for cancer treatment. The nanoparticles include a glyceryl monooleate or monolinoleate (or other mono fatty acid ester); a chitosan; and a cancer therapeutic agent.

In microfluidics, hydrogels are used as valves: When submitted to appropriate stimuli, they swell in a reversible manner and thus can be used to block and open microchannels. However, swelling and unswelling creates a strain at the interface between the gel and the carrier to which it is glued. Repeated use degrades the quality of the valve efficiency.

In actuation, it is usual to have two gels of different nature adhere and through stimulation of one gel, provoke deformation of the assembly. Here again, failures appear at the interface when submitted to deformation.

Soft biological tissues although incomparably more complex, mechanically and osmotically, resemble gels in many respects. In the medical and chirurgical field, for many applications, it is desirable to have two biological tissues adhere to one another, or to have a synthetic gel material (patch, dressing, implant, prosthesis, amniocentesis) adhere to a biological tissue. Adhesion should be sufficient until natural tissue repair occurs through cell colonization or to allow suturing. Adhesion should also be obtained without forming scars. Surgical adhesives known to date are numerous and of varied origin, but not deprived of inconvenient (Duarte A.P. et al., Progress in Polymer Science, 37, 1031-1050 (2012)). Polymer tissue adhesives require complex in vivo control of polymerization or cross-linking reactions and currently suffer from being toxic, weak or inefficient within the wet conditions of the body. Polymer adhesives form films at the interface and often modify greatly the material's permeability and the mechanical properties of the assembly.

In the field of food chemistry, the question of assembling compositions of matter is also important. The assembly must be comestible and gluing of different materials should be effective but discreet. No satisfying solution of general application for gluing or creating adhesion or increasing adhesion between edible gels exists today. Nanoparticles have been used in the field of food chemistry for many applications and are therefore generally considered as safe for application to humans.

It is known to use gluing compositions comprising polymers for gluing synthetic gels. It is also known to use synthetic or natural polymer materials as surgical glue. Some adhesive compositions comprising known adhesive polymers, said compositions may comprise nanoparticles as active principles or as filler in order to increase the fracture resistance of the assembly without leading to an increase in the viscosity of the adhesive (Johnsen, B. B., A. J. Kinloch, et al. (2007). "Toughening mechanisms of nanoparticle-modified epoxy polymers." Polymer 48(2): 530-541. ; Kinloch, A. J. (1987). Adhesion and Adhesives: Science and Technology, Springer;Kendall, K. Molecular Adhesion and Its Applications, Plenum Publishing Corporation, 2001). However, in these prior art compositions, nanoparticles were present

as a minor component as compared to the adhesive agent, and it has never been mentioned or suggested that nanoparticles dispersions or powders themselves could act as adhesive agent between two materials, among which, one at least, is a hydrogel.

For example, US2012/0220911 discloses glass micro particles based on a glass former and at least one trace element (Ag, Fe, Cu, F, etc...) for their use in surgical glues. However, such micro particles are formulated with an adhesive, and their function is to promote wound healing. Gent et al. (Gent, A. N., Hamed, G. R., & Hung, W. J.. Adhesion of elastomer layers to an interposed layer of filler particles. The Journal of Adhesion, 79(10), 905-913 (2003)) showed that powdering self-adhesive elastomers with carbon black particles can increase the self- adhesion energy by a factor of two. However, it has never been mentioned or suggested that nanoparticles powders could act as adhesive agent between two materials, among which, one at least, is a hydrogel. Adhesion in the presence of water is notoriously difficult to achieve (Lee, B. P., Messersmith, P. B., Israelachvili, J. N., & Waite, J. H.. Mussel-Inspired Adhesives and Coatings. Annual Review of Materials Research, 41, 99-132 (2011)).

There remained the need of a gluing solution for gels which would be applicable to gels of synthetic nature and of biological origin, that would be easy to use, cheap, which could provide an adhesion of satisfying resistance (at least superior to the limit of resistance of the gel itself), and of esthetic appearance. Also ideally adhesive junction should not compromise mechanical and osmotic responsiveness of hydrogels especially when assemblies of two hydrogels are made.

## SUMMARY OF THE INVENTION

The object of the present invention is to alleviate at least partly the above mentionned drawbacks.

In the description and the claims, the phrase "consists essentially of' limits the object that follows to the specified materials or steps and to those that do not materially affect the basic and novel characteristic(s) of the invention.

The invention is directed to the use of a composition of inorganic nanoparticles as adhesive, or gluing agent or adhesion promoting agent or adhesion creating agent, or adhesion increasing agent between at least one hydrogel and at least one other article, wherein the composition of nanoparticles is selected from a powder and an aqueous suspension, or aqueous dispersion or aqueous solution, of nanoparticles wherein the composition of nanoparticles contains no more than 20% by weight of other gluing agent as compared to the weight of the dry matter of the composition.

The invention is directed to a composition of nanoparticles, for its medical or surgical use as gluing agent or adhesive for adhering one biological tissue and at least one other article.

The invention is directed to a composition of nanoparticles, for its medical or surgical use as gluing agent or adhesive for adhering one biological tissue and one second biological tissue.

More particularly, the invention is directed to the use of nanoparticles as adhesive or gluing agent or adhesion promoting agent or adhesion creating agent, or adhesion increasing agent, wherein the nanoparticles are located at the interface between the hydrogel and at least one other article.

The invention is notably directed to the use of solid nanoparticles as adhesive, or gluing agent or adhesion promoting agent or adhesion creating agent, or adhesion increasing agent between at least one hydrogel and at least one other article.

More particularly, the invention aims to provide a method of gluing or adhering at least one hydrogel to at least one other article, of an identical or different material, in a simple, efficient and cheap manner. The invention aims to provide a method which is applicable to a wide variety of hydrogels, of synthetic or natural origin. The method according to the invention aims to provide an assembly which is resistant to external stress, notably, the adhesion of the hydrogel to the other article is superior or equal to the limit of resistance of the gel itself. Gluing by nanoparticles is surprising since powdering surfaces with micron-sized particles such as talc provides a standard means of preventing self-adhesion.

This object is preferably achieved with a composition of nanoparticles which is used for adhering or gluing or creating adhesion or increasing adhesion between at least one hydrogel and at least one other article, wherein the nanoparticles represent from 10 to 100% by weight of the dry matter of the composition.

This object is achieved with a composition of nanoparticles which is used for adhering or gluing or creating adhesion or increasing adhesion between at least one hydrogel and at least one other article, wherein the nanoparticles composition is selected from a powder and an aqueous dispersion, an aqueous suspension, or an aqueous solution of nanoparticles.

According to a favorite variant, the invention is directed to the use of an aqueous suspension or aqueous dispersion or aqueous solution of inorganic nanoparticles as adhesive or as gluing agent or adhesion promoting agent or adhesion creating agent, or adhesion increasing agent between a hydrogel and another article wherein the composition of nanoparticles contains no more than 20% by weight of other gluing agent as compared to the weight of the dry matter of the composition.

According to one embodiment, the invention is directed to the use of nanoparticles as adhesive or gluing agent or adhesion promoting agent or adhesion creating agent, or adhesion increasing agent between a hydrogel and another

article wherein both the hydrogel and the other article are distinct from a biological tissue.

According to one embodiment, the invention is directed to the use of nanoparticles as adhesive or gluing agent or adhesion promoting agent or adhesion creating agent, or adhesion increasing agent between a hydrogel and another article wherein at least one of the hydrogel and the other article is distinct from a biological tissue.

According to one embodiment, the invention is directed to the use of nanoparticles as adhesive or gluing agent or adhesion promoting agent or adhesion creating agent, or adhesion increasing agent between a hydrogel and another article wherein one of the hydrogel and the other article is distinct from a biological tissue. Said use is a medical use or a surgical use or a cosmetic use.

According to one embodiment, the invention is directed to the use of nanoparticles as adhesive or gluing agent or adhesion promoting agent or adhesion creating agent, or adhesion increasing agent between a synthetic hydrogel and the skin. Said use is a cosmetic use or a dermatological use.

According to one embodiment, the invention is directed to the use of nanoparticles as a gluing agent or adhesion promoting agent or adhesion creating agent, or adhesion increasing agent between a hydrogel and another article wherein both the hydrogel and the other article are a biological tissue. Said use is a medical use or surgical use.

Additionally, the invention finds application in a method for adhering or gluing or creating adhesion or increasing adhesion between at least one hydrogel and at least one other article, wherein said method comprises:

a- applying a composition of nanoparticles on at least one face of the hydrogel
b- applying the face of the hydrogel bearing the nanoparticles to the article,

and wherein in case the at least one hydrogel or the at least one other article is a biological tissue, it is an isolated tissue or a cultured tissue.

The invention finds application in a method for adhering or gluing or creating adhesion or increasing adhesion between at least one hydrogel and at least one other article, wherein said method consists essentially of:

a- applying a composition of nanoparticles on at least one face of the hydrogel
b- applying the face of the hydrogel bearing the nanoparticles to the article

and wherein in case the at least one hydrogel or the at least one other article is a biological tissue, it is an isolated tissue or a cultured tissue.

Additionally, the invention finds application in a method for adhering or gluing or creating adhesion or increasing adhesion between at least one hydrogel and at least one other article, wherein said method comprises:

a- applying a composition of nanoparticles on at least one face of the article
b- applying the face of the article bearing the nanoparticles to the hydrogel,

and wherein in case the at least one hydrogel or the at least one other article is a biological tissue, it is an isolated tissue or a cultured tissue.

The invention finds application in a method for adhering or gluing or creating adhesion or increasing adhesion between at least one hydrogel and at least one other article, wherein said method consists essentially of:

a- applying a composition of nanoparticles on at least one face of the article
b- applying the face of the article bearing the nanoparticles to the hydrogel

and wherein in case the at least one hydrogel or the at least one other article is a biological tissue, it is an isolated tissue or a cultured tissue.

The invention finds application in a method for adhering or gluing or creating adhesion, or increasing adhesion between at least one hydrogel and at least one other article, wherein said method comprises:

a- applying a composition of nanoparticles on at least one face of the hydrogel a'- applying a composition of nanoparticles on at least one face of the article
b- applying the face of the hydrogel bearing the nanoparticles to the face of the article bearing the nanoparticles

and wherein in case the at least one hydrogel or the at least one other article is a biological tissue, it is an isolated tissue or a cultured tissue.

The invention finds application in a method for adhering or gluing or creating adhesion, or increasing adhesion between at least one hydrogel and at least one other article, wherein said method consists essentially of:

a- applying a composition of nanoparticles on at least one face of the hydrogel a'- applying a composition of nanoparticles on at least one face of the article
b- applying the face of the hydrogel bearing the nanoparticles to the face of the article bearing the nanoparticles,

and wherein in case the at least one hydrogel or the at least one other article is a biological tissue, it is an isolated tissue or a cultured tissue.

The invention finds application in a cosmetic method for adhering or gluing or creating adhesion or increasing adhesion between at least one hydrogel and the skin or the nails, wherein said method comprises:

a- applying a composition of nanoparticles on at least one face of the hydrogel
b- applying the face of the hydrogel bearing the nanoparticles to the skin or the nails.

The invention finds application in a cosmetic method for adhering or gluing or creating adhesion or increasing adhesion between at least one hydrogel and the skin or the nails, wherein said method consists essentially of:

a- applying a composition of nanoparticles on at least one face of the hydrogel
b- applying the face of the hydrogel bearing the nanoparticles to the skin or the nails.

The invention finds application in a cosmetic method for adhering or gluing or creating adhesion or increasing adhesion between at least one hydrogel and the skin or the nails, wherein said method comprises:

a- applying a composition of nanoparticles on the skin or the nails
b- applying the hydrogel to the part of the skin or the nails bearing the nanoparticles.

The invention finds application in a cosmetic method for adhering or gluing or creating adhesion or increasing adhesion between at least one hydrogel and the skin or the nails, wherein said method consists essentially of:

a- applying a composition of nanoparticles on the skin or the nails
b- applying the hydrogel to the part of the skin or the nails bearing the nanoparticles.

The invention finds application in a cosmetic method for adhering or gluing or creating adhesion, or increasing adhesion between at least one hydrogel and at least one other article, wherein said method comprises:

a- applying a composition of nanoparticles on at least one face of the hydrogel a'- applying a composition of nanoparticles on the skin or the nails
b- applying the face of the hydrogel bearing the nanoparticles to the face of the skin or the nails bearing the nanoparticles.

The invention finds application in a cosmetic method for adhering or gluing or creating adhesion, or increasing adhesion between at least one hydrogel and at least one other article, wherein said method consists essentially of:

a- applying a composition of nanoparticles on at least one face of the hydrogel
a'- applying a composition of nanoparticles on at least one face of the the skin or the nails
b- applying the face of the hydrogel bearing the nanoparticles to the face of the skin or the nails bearing the nanoparticles.

Additionally, the invention finds application in a medical or surgical method for adhering or gluing or creating adhesion or increasing adhesion between at least one biological tissue and at least one other article, wherein said method comprises:

a- applying a composition of nanoparticles on at least one face of the biological tissue
b- applying the face of the biological tissue bearing the nanoparticles to the article.

Additionally, the invention finds application in a medical or surgical method for adhering or gluing or creating adhesion or increasing adhesion between at least one biological tissue and at least one other article, wherein said method comprises:

a- applying a composition of nanoparticles on at least one face of the article

b- applying the face of the article bearing the nanoparticles to the biological tissue.

Additionally, the invention finds application in a medical or surgical method for adhering or gluing or creating adhesion or increasing adhesion between at least one first biological tissue and at least one second biological tissue, wherein said method comprises:

a- applying a composition of nanoparticles on at least one face of the first biological tissue
b- applying the face of the first biological tissue bearing the nanoparticles to the second biological tissue.

Additionally, the invention finds application in a medical or surgical method for adhering or gluing or creating adhesion or increasing adhesion between at least one biological tissue and at least one other article, wherein said method consists essentially of:

a- applying a composition of nanoparticles on at least one face of the biological tissue
b- applying the face of the biological tissue bearing the nanoparticles to the article.

Additionally, the invention finds application in a medical or surgical method for adhering or gluing or creating adhesion or increasing adhesion between at least one biological tissue and at least one other article, wherein said method consists essentially of:

a- applying a composition of nanoparticles on at least one face of the article
b- applying the face of the article bearing the nanoparticles to the biological tissue.

Additionally, the invention finds application in a medical or surgical method for adhering or gluing or creating adhesion or increasing adhesion between at least one first biological tissue and at least one second biological tissue, wherein said method consists essentially of:

a- applying a composition of nanoparticles on at least one face of the first biological tissue
b- applying the face of the first biological tissue bearing the nanoparticles to the second biological tissue.

Additionally, the invention finds application in a medical or surgical method for adhering or gluing or creating adhesion or increasing adhesion between at least one biological tissue and at least one other article, wherein said method comprises:

a- applying a composition of nanoparticles on at least one face of the biological tissue,
a'- applying a composition of nanoparticles on at least one face of the article,
b- applying the face of the biological tissue bearing the nanoparticles to the face of the article bearing the nanoparticles.

Additionally, the invention finds application in a medical or surgical method for adhering or gluing or creating adhesion or increasing adhesion between at least one first biological tissue and at least one second biological tissue, wherein said method comprises:

a- applying a composition of nanoparticles on at least one face of the first biological tissue
a'- applying a composition of nanoparticles on at least one face of the second biological tissue,
b- applying the face of the first biological tissue bearing the nanoparticles to the face of the second biological tissue bearing the nanoparticles.

Additionally, the invention finds application in a medical or surgical method for adhering or gluing or creating adhesion or increasing adhesion between at least one biological tissue and at least one other article, wherein said method consists essentially of:

a- applying a composition of nanoparticles on at least one face of the biological tissue,
a'- applying a composition of nanoparticles on at least one face of the article,
b- applying the face of the biological tissue bearing the nanoparticles to the face of the article bearing the nanoparticles.

Additionally, the invention finds application in a medical or surgical method for adhering or gluing or creating

adhesion or increasing adhesion between at least one first biological tissue and at least one second biological tissue, wherein said method consists essentially of:

a- applying a composition of nanoparticles on at least one face of the first biological tissue
a'- applying a composition of nanoparticles on at least one face of the second biological tissue,
b- applying the face of the first biological tissue bearing the nanoparticles to the face of the second biological tissue bearing the nanoparticles.

The invention gives access to an assembly of at least one hydrogel and at least one other article, wherein the interface between the at least one hydrogel and the at least one other article is a layer (including monolayer and multi-layers) of nanoparticles, and wherein in case the at least one hydrogel or the at least one other article is a biological tissue, it is an isolated tissue or a cultured tissue.

The invention also gives access to an assembly of at least one hydrogel and at least one other article, wherein the interface between the at least one hydrogel and the at least one other article consists essentially of a layer (including monolayer and multi-layers) of nanoparticles, and wherein in case the at least one hydrogel or the at least one other article is a biological tissue, it is an isolated tissue or a cultured tissue.

The invention also gives access to a kit for gluing a hydrogel to an article, wherein said kit comprises at least a hydrogel and at least a composition of nanoparticles.

The invention also gives access to a surgical kit, wherein it comprises at least one composition of nanoparticles and at least one article chosen from: a suturing needle, a suturing strip, suturing staples, a prosthesis, a mesh.

Preferred embodiments comprise one or more of the following features:
The composition of nanoparticles is an aqueous suspension or an aqueous dispersion or an aqueous solution of nanoparticles.

The composition of nanoparticles is a powder.

A composition of nanoparticles, wherein the nanoparticles are selected from: clays, silicates, alumina, silica, kaolin, carbonaceous nanoparticles, grafted carbon nanotubes, hydroxyapatite, magnetic nanoparticles, metal oxides, noble metals, quantum dots.

A composition of nanoparticles, wherein the nanoparticles have an average particle size from 1 nm to 1000 nm, preferably from 2 nm to 500 nm even more preferably from 5 nm to 300 nm.

A composition of nanoparticles, wherein the hydrogel is of a material selected from: poly(acrylamide) derivatives, PDMA (poly(N,N-dimthylacrylamide)), poly-NIPAM (poly(N-isopropylacrylamide)), a synthetic or extracted gel based on proteins, a synthetic or extracted gel based on polysaccharides, poly(ethylene glycol hydrogel) (PEG), poly(vinyl alcohol) hydrogels, block copolymers of PEG and hydrophobic polyesters.

A composition of nanoparticles, for use as above disclosed wherein the hydrogel is a biological tissue.

A composition of nanoparticles, for use as above disclosed wherein the other article is of a material selected from: a hydrogel, a glass, a polymer, a biological tissue.

The invention finds application in a method for adhering or gluing or creating adhesion or increasing adhesion between a hydrogel and another article, wherein step b is achieved underwater.

The invention also finds application in any of the methods as above disclosed, wherein said method comprises a preliminary step (before step a) of measuring the nanoparticles adsorption at the hydrogel's surface.

The invention also finds application in any of the methods as above disclosed, wherein said method comprises a preliminary step (before step a) of selecting nanoparticles capable of adsorption at the hydrogel's surface.

According to a favorite embodiment, the nanoparticles should be capable of adsorbing at the hydrogel's surface in an amount of at least 0.1 $mg/m^2$.

According to a favorite embodiment, the nanoparticles should be capable of adsorbing at the hydrogel's surface in an amount of between 0.1 $mg/m^2$ and 10 $g/m^2$.

The invention also finds application in any of the methods as above disclosed, wherein said method comprises a preliminary step (before step a) of measuring the nanoparticles adsorption at the other article's surface.

The invention also finds application in any of the methods as above disclosed, wherein said method comprises a preliminary step (before step a) of selecting nanoparticles capable of adsorption at the other article's surface.

According to a favorite embodiment, the nanoparticles should be capable of adsorbing at the other article's surface in an amount of at least 0.1 $mg/m^2$.

According to a favorite embodiment, the nanoparticles should be capable of adsorbing at the other article's surface in an amount of between 0.1 $mg/m^2$ and 10 $g/m^2$.

The invention finds application in a method comprising a step of applying a composition of nanoparticles on at least one face of the other article after applying a composition of nanoparticles on at least one face of the hydrogel (step a) and before applying the face of the hydrogel bearing the nanoparticles to the article (step b).

The invention finds application in a method wherein adhesion is achieved at a temperature inferior or equal to

40°C.

The invention finds application in a method wherein adhesion is achieved at a temperature inferior or equal to room temperature.

The invention finds application in a method wherein adhesion is achieved at a temperature inferior or equal to body temperature.

The invention finds application in a method comprising a step of drying the hydrogel between applying a composition of nanoparticles on at least one face of the hydrogel (step a) and applying the face of the hydrogel bearing the nanoparticles to the article (step b).

The invention finds application in a method comprising a step of applying pressure to the assembly of the hydrogel and the other article during or after step b (applying the face of the hydrogel bearing the nanoparticles to the article and/or applying the face of the article bearing the nanoparticles to the hydrogel).

The invention finds application in a cosmetic method for gluing a hydrogel to the skin or to the nails.

The invention gives access to an assembly of a hydrogel and another article, wherein the concentration of nanoparticles at the interface is from 0.1 mg/$m^2$ to 10 g/$m^2$.

The invention gives access to an assembly of a hydrogel and another article, wherein the adhesion of the hydrogel to the other article is superior to the hydrogel's resistance and is superior to the other article's resistance.

The invention gives access to an assembly of a hydrogel and another article, wherein it is selected from: a microfluidic equipment, a biochip, a gel permeation equipment, an actuation gel assembly, an edible gel assembly, a cosmetic or pharmaceutical patch or dressing, a biosensor, a medical electrode, a contact lens, an implant, a prosthesis, a surgical strip.

The invention gives access to a kit for gluing a hydrogel to the skin or to an organ.

The invention gives access to a kit which is a cosmetic or dermatological kit.

The invention gives access to a kit for gluing a hydrogel to the skin wherein the hydrogel is part of medical electrodes.

The invention gives access to a kit for gluing a hydrogel to the skin or to an organ wherein the hydrogel is part of a prosthetic device.

The invention gives access to a kit which comprises a hydrogel with nanoparticles adsorbed on at least one of its faces.

Further features and advantages of the invention will appear from the following description of embodiments of the invention, given as non-limiting examples, with reference to the accompanying drawings listed hereunder.

## BRIEF DESCRIPTION OF THE DRAWINGS

Fig. 1 shows a schematic representation of the formation of an assembly of two hydrogels with a layer of nanoparticles between the two gels. Nanoparticle act as connectors between the two adherents and are defined as the gluing agent.

Fig. 2 shows graphics illustrating lap-shear adhesion tests.

Figure 2a includes a comparison of force (ordinate, in N) - displacement (abscissa, in mm) curves for PDMA S0.1 ribbon (continuous line) and for the lap-joint glued by spreading of 15 μL of TM-50 silica solution (doted line). Displacement is measured by optical extensometer from two markers, initially spaced by 20 mm and centered on the joint. The ribbons were *w* = 5mm wide and *h*=2mm thick. The overlap length is *l*=10mm The PDMA S0.1/S0.1 assemblies break outside the joint.

Figure 2b illustrates a lap-shear test yielding quantitative measurement of adhesion energy of glued PDMA S01 gels. The experiment was repeated three times. Figure 2b is a graph representing normalized force (ordinate in N/m) - strain (abscissa) curves for PDMA S0.1/S0.1 lap-joint with the overlap length of *l*=5 mm glued with 6 μL of TM-50 silica solution. Adhesive failure by interfacial peeling was observed. All gel ribbons were cut from the same gel plate and the tensile modulus was measured to be $E \approx 8.1 \pm 1.0$kPa. In these tests *w*=2mm and *h*=5mm.

Fig. 3 shows graphics illustrating lap-shear adhesion tests of PDMA gels with different crosslinking densities (0.1mol%, 0.5 mol.% and 1.5 mol.%) glued by TM-50 particles.

Figure 3a is a graph representing normalized force (ordinate, in N.$m^{-1}$) - strain (abscissa) curves for lap joint with overlap length of 10 mm: S0.1/S0.1 (● line), S0.5/S0.5 (□ line) and S1.5/S1.5 (■ line) PDMA gel assemblies. Fracture occured outside the joint for all assemblies (*w*=5mm and *h*=2mm). Strain is calculated from the optical extensometer.

Figure 3b is a graph representing normalized force (ordinate, in N.$m^{-1}$) - strain (abscissa) curves for lap joint with overlap length of 5 mm: S0.1/S0.1 (● line), S0.5/S0.5 (□ line) and S1.0/S1.0 (solid line) PDMA gel assemblies. Adhesive interfacial fracture in the joint occurred for assemblies S0.5/S0.5 and S1.0/S1.0 whereas bulk fracture occurred outside the joint for S01/S01 assembly (*w*=5mm and *h*=2mm).

Figure 3c is a graph representing stress on the left axis (ordinate in kPa) and Force on the right axis (ordinate in N) as a function of strain (abscissa) and illustrates the effect of cross-linking density on the gel tensile behaviour: S0.1 (continuous line), S0.5 (long dashed line) and S1.5 (short dashed line). Strain is calculated from optical extensometer and stress is defined as engineering stress.

Fig. 4 shows force (ordinate, in N) - displacement (abscissa, in mm) curves for PDMA S0.1/S0.1 lap-joint with the overlap length of 10 mm, glued with 15μL of TM-50 silica solution. All gel ribbons had w=5 mm and h=2mm and were cut from the same gel plate. Bulk failure outside the joint was systematically observed.

Fig. 5 illustrates water resistant adhesion and energy dissipation. Figure 5a is a graphic representing lap-shear test for PDMA S0.1/S0.1 assembly glued with TM-50 solution at the preparation state, $Q_0$ (● line) and after swelling in water for 3 days, $Q_e$ (○ line) and attaining maximum equilibrium swelling $Q_e$. Force in Newton (ordinate, N) is represented as a function of displacement (abscissa, mm). Initially (at preparation state) the lap joint dimensions were *l*=5mm *w*=5mm and *h*=2mm. 6μL of solutions was spread to make the junction.

Figure 5b is a scanning electron micrograph showing the presence of adsorbed silica layer, which persisted after multiple washing and soaking of the S0.1 gel surface in water for several days.

Figure 5c is a graph representing adhesion energy (ordinate, in $J.m^{-2}$) - swelling degree (abscissa) curves of joints made of PDMA S0.1 hydrogels swollen prior to being glued with AL-30 silica solutions to various degrees of swelling Q (●) and adhesion energy of joints made of S0.1 hydrogels glued with TM-50 silica solutions at as-synthesized swelling degree ($Q_0 \approx 8.5$) and after being immersed in water and swollen to reach the maximum, equilibrium swelling degree, $Q_e \approx 41$ (▲).

Figure 5d is a graph representing lap-shear test for PDMA S0.1/S0.1 glued by spreading 6μL droplet of HS-40 silica solution (continuous line). After adhesive interfacial failure by peeling the joint was repaired by bringing ribbons back to contact and pressing with fingers for a dozen of seconds. The joint recovered its strength (dashed line). The lap joints were 5 mm wide, 2 mm thick and the overlap length was 5 mm.

Fig. 6 illustrates lap-shear test for PDMA S0.1 gels glued by various particle solutions. In figure 6, force (ordinate, N/m) is represented as a function of displacement (abscissa, mm). In order of increasing deformation at break: adhesive failure by interfacial peeling occured for CNT-Thy (× line), CNC1 (dotted line), SM-30 (○ line), and HS-40 (▲ line), and the fracture outside the junction occurred for TM-50 (●) and AL-30 (continuous line). Lap joint dimensions were *l*=5mm *w*=5mm and *h*=2mm. 6μL of solutions was spread to make the junction.

Figure 7 illustrates tensile test of lap-joints made of gels of different stiffness or chemical nature. Figure 7a: Lap-shear force (ordinate in N) - displacement (abscissa, in mm) curve for an assembly made of soft PDMA S0.1 and rigid PDMA S1.5 (▲) gels. For comparison the results obtained under identical conditions for the symmetric PDMA S0.1/S0.1 assembly are plotted (●). Lap joint dimensions were *l*=10mm *w*=5mm and *h*=2mm. 15μL of TM-50 solution was spread to make the junction. Figure 7b, Lap-shear force (ordinate in N) - displacement (abscissa, in mm) curve for gelatin and S0.1 PDMA gel assembly glued with TM-50 silica solution (★). The S0.1 and gelatin gel had different rigidity.

Silica nanoparticles enable one to glue the gels of different chemical natures and of different mechanical properties. For both PDMA S0.1/PDMA S0.5 and PDMA S0.1/gelatin assemblies, the failure occurred outside the lap joint and cracks propagated in tension mode.

Figure 8 illustrates how junctions glued by cellulose nanocrystals CNC1 are self-repairable and repositionable. Figure 8 is a graph showing lap-shear force (ordinate in N) - displacement (abscissa, in mm) curve for PDMA S0.1/S0.1 glued by spreading 6μL droplet of CNC1 solution (continuous line). After adhesive interfacial failure by peeling, the joint was repaired by putting ribbons back to contact and pressing with fingers for a dozen of seconds. The joint recovered its strength (dashed line). There is no need to re-apply glue. The lap joints were 5 mm wide, 2 mm thick and the overlap length was 5 mm.

Fig. 9 illustrates lap-shear adhesion tests. Figure 9a is a graph representing the normalized failure force (ordinate, in $N.m^{-1}$) measured in lap-shear adhesion test for lap joints of various overlap length / (abscissa, in mm) made of S0.1 gels ribbons glued by 15μL of TM-50 silica solutions. Circles correspond to failure by fracture outside the joint and triangles to adhesive failure by peeling at the interface. Ribbons were 5 mm wide and 2 mm thick. Figure 9b: is a graph representing normalized failure force (ordinate in N/m) measured in lap-shear adhesion test for lap joints glued using solutions of various particles: carbon nanotubes CNT-Thy (A), cellulose nanocrystals CNC1 (B), and silica of various sizes SM-30 (C), HS-40 (D), TM-50 (E), and AL-30 (F). For particle solutions E and F fracture occurred outside the joint, for A, B, C, and D adhesive failure by peeling was observed. The lap joints were 5 mm wide, 2 mm thick and the overlap length was 5 mm. Figure 9c is a graph representing adhesion energy $G_{adh}$ (unfilled bars, ordinate in $J/m^2$) measured in lapshear test and fracture energy $G_c$ (striped bars, ordinate in $J/m^2$) measured in single edge notch tensile test of PDMA gels of various cross-linking densities 0.1mol% (S0.1), 0.5 mol% (S0.5), and 1 mol% (S1.0). Lap joints were glued with $0.3\mu L/mm^2$ of TM-50 silica solutions and lap joint dimension were l=5mm, w=5mm and h=2mm for S0.5 and S1.0 and l=2mm, w=5mm and h=2mm for S0.1.

Figure 10 is a graph representing normalized force (ordinate in N/m) - displacement (abscissa, in mm) curves for lap-joints made of ribbons cut from calf liver and glued by spreading 60μL of TM-50 silica solution and pressing ribbons with a finger for 30 seconds. The ribbons were cut with a scalpel blade and no treatment was applied to liver surfaces before gluing. The moduli of two livers are respectively 15.0±1.7 kPa and 12±1.5 kPa. The ribbons were 10 mm wide, 3 mm thick and the overlap length was 20 mm. Results for two livers are presented.

Figure 11 is a drawing which schematically illustrates the single notch fracture test method.

Figure 12 is a photograph of a PDMA S0.1/S1.5 assembly after immersion in water. Glued at their preparation state by TM-50 solution, both PDMA S0.1 and PDMA S1.5 gels had initially the same size (diameter of about 10 mm). Picture shows gels after 5 hours of swelling in deionised water. Highly cross-linked PDMA S1.5 gel (top piece) is less swollen than PDMA S0.1 gel (bottom piece). Gel S0.1 shows a fivefold over-swelling when immersed in water, whereas the more tightly cross-linked gel S1.5 over-swells by a factor of 1.7 only. As a result, interfacial stresses induced by heterogeneous over-swelling exceed considerably shear stresses applied in mechanical lap-shear tests. Hence, for S0.1/S1.5 assemblies, interfacial failure was observed after more than 5 hours of immersion and over-swelling in water. Still adhesion joint held for quite a long time and the de-bonding was slow.

Fig. 13 is a FTIR-ATR spectra showing the remaining adsorbed silica particles onto a S0.1 gel surface at Qo (long dashed line) and $Q_e$ (short dashed line) after soaking and washing in water. Dried silica suspension (○ line) and S0.1 dried gel (solid line) are presented as guideline.

Fig. 14: Scanning Electron Microscopy (SEM) of adsorbed silica particles onto a S0.1 gel surface at $Q_0$ (figure 14a) and $Q_e$ (figure 14b), after water soaking and washing. Samples were dried prior observation.

Fig.15 is a graph representing normalized force (ordinate in N/m) - volume of silica suspension (abscissa, in μl) results for lap-joints made of PDMA S0.1 and glued by spreading TM-50 silica solution and applying to ribbons a pressure of 10kPa for 30 seconds. The ribbons were 5 mm wide, 2 mm thick and the overlap length was 15 mm.

Fig. 16 is a graph representing spectrum intensity measured at 1100 $cm^{-1}$ and normalized by intensity measured at 1400 $cm^{-1}$ (ordinate) - volume of AL-300 silica suspension (abscissa, in μl) results of FTIR-ATR studies of adsorption of AL-300 silica suspensions onto PDMA M0.1 gel surfaces. The droplets were spread on 10mm x 5mm surface of as synthesized gels.

Fig. 17 is a graph representing tensile force (ordinate, in N) - strain (abscissa) curves for lap joint made by gluing PDMA M0.1 gels. The joints overlap length was 20 mm their width 5 mm and the thickness of gel ribbons was about 2 mm. The dashed curve shows a typical result for M01/M01 junction and quantifies self-adhesion strength of these gels. Continuous curves are obtained for PDMA M0.1 ribbons glued to a M0.1 gel coated with an adsorbed layer of AL30 silica nanoparticles. The adsorbed layer was obtained by spreading 5 μl of silica solution onto 20mm x 5mm surface of the gel and subsequent washing it in deionized water. To make lap joints the ribbons were brought into contact for 5 minutes.

Fig 18 is a graph representing tensile force (ordinate, in N) - strain (abscissa) curves for lap joint made by gluing PDMA M0.1 gels. The joints overlap length was 20 mm their width 5 mm and the thickness of gel ribbons was about 2 mm. The dashed curve shows a typical result for M01/M01 junction and quantifies self-adhesion strength of these gels. Continuous and dotted curves are obtained for PDMA M0.1 ribbons glued by spreading of 5 μl of silica AL-30 and PEGolated silica JL-100 solutions, respectively. To make lap joints the ribbons were brought into contact for 5 minutes.

## DETAILED DESCRIPTION OF THE INVENTION

The invention is directed to the use of a composition of inorganic nanoparticles, wherein the composition of nanoparticles is selected from a powder and an aqueous suspension, or aqueous dispersion or aqueous solution, of nanoparticles, wherein the composition of nanoparticles contains no more than 20% by weight of other gluing agent as compared to the weight of the dry matter of the composition, for gluing at least one hydrogel to at least one other article.

According to the present invention, by "gluing", it is also meant "adhering", "bonding", "adhesive bonding" or "fixing by an adhesive".

The invention is directed to the use of nanoparticles as gluing agent between at least one hydrogel and at least one other article.

According to the present invention, by "gluing agent", it is also meant "adhesive", "adhesion creating agent", "adhesion increasing agent", "bonding agent", "glue", "adhesion promoter".

According to the invention, in conformity with the skilled professional's knowledge, a gluing agent is a material located or introduced at the interface between two articles, distinct from these articles, that creates, yields, promotes or increases adhesion strength and/or adhesion energy between said articles.

To the skilled professional, adhesion strength is known as the measure of stresses necessary to induce a failure of the bonded junction. Adhesion strength is expressed as the maximal stress in kPa.

To the skilled professional, adhesion energy is known as the mechanical energy necessary to propagate a crack within the glued interface. Adhesion energy is expressed as an energy per glued surface in $J/m^2$.

Different test methods exist for measuring adhesion, some measuring adhesion strength, others measuring adhesion energy, the method to be used being selected according to the type of material assembled in the adhesion process (Kendall, K. Molecular Adhesion and Its Applications, Plenum Publishing Corporation, 2001; Kinloch, A. J., Adhesion and Adhesives: Science and Technology, London, Chapman and Hall, 1987).

Preferably, the adhesion is measured by a lap-shear test according to the protocol disclosed in the experimental part. Preferably, the adhesion of the hydrogel to the other article is superior to the hydrogel's resistance and to the other article's resistance. It means that, in this test, failure occurs outside the lap joint. However good adhesion but interfacial failure or peeling can occur in some cases.

According to the invention, the hydrogel and the other article exist before they are adhered to one another. The composition of nanoparticles also is distinct from these objects until it is applied at the interface between them.

The composition of nanoparticles, when it is applied at the interface, does not significantly modify the bulk properties of the hydrogel and/or the other article. Notably, it does not significantly modify the bulk mechanical and rheological properties of the gel, like Young's modulus.

- Composition of nanoparticles:

As is clearly explained all along the disclosure, in the method and use according to the invention, the composition of nanoparticles is a composition distinct from the hydrogel and, contrary to the prior art is not used in the process of making the hydrogel.

In the description of this invention, suspension or dispersion or solution of nanoparticles have the same meaning.

The term "nanoparticles" means particles from 1 nm to 1000 nm, preferably from 2 to 500 nm and even more preferably from 5 to 300 nm in size. For most nanoparticles, the size of the nanoparticles is the distance between the two most distant points in the nanoparticle. For anisotropic nanoparticles, such as tubes whiskers or cylinders, the size of the diameter is the diameter of the smallest cylinder in which the nanoparticle is inscribed. Nanoparticle size can be determined by different methods such as Dynamic Light Scattering (DLS), Small Angle X-ray Scattering (SAXS), Scanning Mobility Particle Sizer (SMPS), Scanning Electron Microscopy (SEM), Transmission Electron Microscopy (TEM) (Orts-Gil, G., K. Natte, et al. (2011), Journal of Nanoparticle Research 13(4): 1593-1604; Alexandridis, P. and B. Lindman (2000), Amphiphilic Block Copolymers: Self-Assembly and Applications, Elsevier Science; Hunter, R. J. and L. R. White (1987). Foundations of colloid science, Clarendon Press.).

Preferably, nanoparticles are selected among solid nanoparticles.

The compositions, preferably the aqueous suspensions of nanoparticles which can be used according to the invention can comprise nanoparticles of different chemical nature, of different sizes, and/or of different shapes.

The nanoparticles can be in the form of a sphere, needle, flake, platelet, tube, fiber, cube, prism, whiskers or have an irregular shape.

Nanoparticles include without limitation the nanofibrils, nanochips, nanolatexes, nanotubes, expandable nanoparticles.

Among the mineral nanoparticles, one can mention metal oxides, alumina, silica, kaolin, hydroxyapatite, calcium carbonate, silicates such as micas quartz, zeolites or clays such as hectorite, laponite, montmorillonite, bentonite, smectite,...

Mineral particles may include, but are not limited to, metal particles. Metal particles encompass particles formed exclusively with metals chosen among alkaline earth metal, transitional metal, rare earth metal, and alloys thereof. In some embodiments, the metal may be aluminum, copper, cadmium, selenium, silver, gold, indium, iron, platinum, nickel, molybdenum, silicon, titanium, tungsten, antimony, palladium, zinc, tin, and alloys thereof. These metal particles may be metal organo modified nanoparticles having chemical entities grafted to their surface or having a self-assembled monolayer of compounds, such as organosulfur compounds, on their surface.

In some embodiments, particles may be particles of metal oxides, such as iron oxides (FeO, $Fe_2O_3$, $Fe_3O_4$) cerium oxide (CeO), alumina ($Al_2O_3$), zirconium oxide ($ZrO_2$), titanium oxide ($TiO_2$), titanates ($BaTiO_3$, $Ba_{0.5}Sr_{0.5}TiO_3$, $SrTiO_3$), indium oxide ($In_2O_3$), tin oxide ($SnO_2$), antimony oxide ($Sb_2O_3$), magnesium oxide (MgO), calcium oxide (CaO), manganese oxides ($Mn_3O_4$, $MnO_2$), molybdenum oxide ($MoO_3$), silica ($SiO_2$), zinc oxide (ZnO), yttrium oxide ($Y_2O_3$), bismuth oxychloride.

Particles may be metal carbides, nitrides, borides, sulphides and hydroxides.

Nanoparticles based on $Fe_2O_3$ have the advantage for in vivo applications that they provide a black colour to the surface wherein they have been applied, therefore, the treated surface can be controlled. The colour can be removed by washing after the biological tissue had rebuilt.

Nanoparticles can have a composite structure like for example $SiO_2$@metal particles (e.g. $SiO_2$@Pd) or core-shell particles (e.g. $Fe_2O_3$core-$SiO_2$ shell or quantum dot core/$SiO_2$shell)).

They can also be organo-metallic nanoparticles: they are metal or metal oxide, carbides, nitrides, borides, sulphides and hydroxides nanoparticles, coated or grafted by an organic material.

Nanoparticles can be selected among metal inorganic salts: Inorganic salts include barium sulfate, calcium carbonate, calcium sulfate, calcium phosphate, magnesium hydrogen carbonate.

Nanoparticles can be selected among metal soaps derived from organic carboxylic acids having from 8 to 22 carbon atoms, preferably from 12 to 18 carbon atoms, for instance zinc stearate, magnesium or lithium stearate, zinc laurate, magnesium myristate.

Nanocomposite particles are included in the scope of the invention like for example core/shell metal/silica nanoparticles.

Nanoparticles can be surfactant or polymer stabilized solid nanoparticles. Polymers can be grafted or adsorbed to the surface of nanoparticles. However, it has been noted that some nanoparticles with polymers grafted or adsorbed on their surface provide less satisfactory adhesion than the original nanoparticles. Checking adorption properties of nanoparticles to the surface of the hydrogel and to the other article permits ensuring the right choice of nanoparticles. This step can be achieved according to methods disclosed in the description and the experimental part.

According to one favorite embodiment, nanoparticles are selected from: clays, silicates, alumina, silica, kaolin, carbonaceous nanoparticles, grafted carbon nanotubes,hydroxyapatite, magnetic nanoparticles, metal oxides, noble metals, quantum dots.

Preferably, the nanoparticles are not thrombin-conjugated.

According to a favorite variant, at least part of the nanoparticles are silica nanoparticles. Advantageously, the aqueous suspension is an aqueous suspension of colloidal silica. Nanoparticles which are used in the invention are selected as a function of the hydrogel's nature. The nanoparticles should be capable of adsorption at the hydrogel's surface. The selection of the appropriate nanoparticles suspension can be achieved by testing the nanoparticles affinity (adsorption) with the hydrogel. Testing methods are disclosed in a detailed manner in the experimental part.

Nanoparticles can additionally be selected as a function of the other article's nature. The nanoparticles should preferably be capable of adsorption at the other article's surface. The selection of the appropriate nanoparticles suspension can be achieved by testing the nanoparticles affinity (adsorption) with the other article by using the same methods.

The skilled professional knows how to modify the surface chemistry of the nanoparticles to optimize interactions and adsorption on a polymer or a tissue and also the surface chemistry of the objects he wants to adhere or glue to a gel or tissue to obtain a good adsorption or anchoring of the particles to this object.

Briefly, a first method rests on Fourier transform infrared spectroscopy coupled with ATR. Attenuated total reflectance (ATR) is a sampling technique used in conjunction with infrared spectroscopy which enables sample surfaces to be examined. The detection and the quantification of adsorbed nanoparticles layer onto the gel surface can be achieved. The proposed method consists in immersing the gel sample into the nanoparticle solution or depositing a droplet of nanoparticle solution on the gel surface, then the gel sample is soaked and washed in a large volume of water during several days. Samples can be dried prior to ATR-FTIR analysis. The presence of an adsorbed nanoparticle layer on the gel surface which persists after soaking enables to select the appropriate nanoparticles to use as adhesive. Conversely, the absence of nanoparticle at the gel surface implies weak adhesive properties of the tested nanoparticles.

Alternately, a second method rests on Scanning Electron Microscopy (SEM) in combination with Energy Dispersive X-ray (EDX). The sample preparation is identical to the one disclosed above for the ATR-FTIR method. EDX is an analytical technique used for the elemental analysis or chemical characterization of a sample. The first micrometers of the surface are probed.

Finally, with the method of thermal isotherms (Hourdet, D. and L. Petit (2010). Macromolecular Symposia. C. S. Patrickios. Weinheim, Wiley-V C H Verlag Gmbh. 291-292: 144-158) the skilled professional can also determine comparatively the nanoparticles best suited for providing adhesion to a gel surface or can carry out thermal isotherms adjusting pH to optimize adsorption mechanism, for example for anionic polyacrylic acid (PAA) macromolecules on the silica surface (Wisniewska, M. (2010) Journal of Thermal Analysis and Calorimetry, 101(2), 753-760. doi:10.1007/s10973-010-0888-4).

The method consists in preparing different series of mixtures of nanoparticle and polymer chains (of the same chemical nature as the gel) by introducing increasing amounts of polymer into the nanoparticle suspension of fixed concentration. The samples are then stored for several days to provide sufficient time to the polymer chains to be adsorbed onto the nanoparticles surface and to equilibrate. The nanoparticles are then settled down by centrifugation and the supernatant is recovered for titration. The total concentration of free polymer chains in the supernatant (Cp) is determined by titration using GPC (Gel Permeation Chromatography) or using a total organic carbon (TOC) analyser or other methods of titrations. The amount of adsorbed polymer on nanoparticle surfaces ($\Gamma$ in $mg/m^2$) can be estimated and is usually plotted against the equilibrium polymer concentration.

To the difference of nanocomposite gels, the compositions of nanoparticles according to the invention are a liquid or a powder.

Advantageously, the nanoparticles are used as an aqueous suspension (or dispersion or solution) of nanoparticles.

According to another embodiment, the compositions of nanoparticles are under powder form.

Aqueous suspensions of nanoparticles are commercially available. One can mention the aqueous suspensions of colloidal silica Ludox® from Grace Davison.

They can be prepared for any of the above-mentioned material by using methods known to the skilled professional Stöber et al. method (Controlled growth of monodisperse silica spheres in the micron size range, Journal of colloid and interface science 26, 62 (1968)).

Advantageously, the aqueous suspension or powder of nanoparticles which can be used according to the invention does not contain any other gluing agent. It means that the aqueous suspension of nanoparticles does not contain a compound known as a gluing agent in a concentration that would allow it to play the function of gluing agent. Among known gluing agents, one can mention:

- Synthetic adhesives: monomers, synthetic polymers (other than polymer nanoparticles), notably cyanoacrylates, urethanes, dendrimers;
- Natural adhesives: fibrin, collagen, gelatin, polysaccharides, thrombian, transglutaminase.

The composition, preferably the aqueous suspension or the powder, of nanoparticles contains no more than 20%, or better, no more than 10% by weight of other gluing agent as compared to the weight of the dry matter of the composition, respectively aqueous suspension, preferably, less than 5% weight, even more preferably less than 2 % and better less than 1%, even better, less than 0,5%.

However, materials distinct from the nanoparticles can be present in the composition, preferably the aqueous suspension or the powder, and notably mineral or organic ions can be present in the composition, preferably the suspension or the powder.

According to the invention, the nanoparticles have the function of gluing agent in the compositions wherein they are present. And in these compositions, the nanoparticles represent from 10 to 100% by weight of the weight of the dry matter of the aqueous suspension or the powder.

Preferably, the nanoparticles represent from 20 to 100% by weight of the weight of the dry matter of the composition, preferably the aqueous suspension or the powder, even more preferably, from 30 to 100%, and advantageously, from 40 to 100%, better from 50 to 100%, even better from 60 to 100%, preferably from 70 to 100%, even better from 80 to 100%, even more preferably from 90 to 100%. According to a favorite variant, the nanoparticles represent from 95 to 100% by weight of the weight of the dry matter of the composition, preferably the aqueous suspension, even better from 98 to 100%, and even more preferably from 99 to 100%.

Preferably, the aqueous suspension of nanoparticles consists essentially of nanoparticles suspended (or dispersed) in water. It means that other components can be present in the suspension, but they do not modify the properties of the suspension in a noticeable manner. Especially, other components can be present in the suspension, but they do not significantly modify the adhesive properties of the suspension.

Alternately, the composition of nanoparticles consists essentially of nanoparticles in powder form. It means that other components can be present in the powder, but they do not modify the properties of the powder in a noticeable manner. Especially, other components can be present in the powder, but they do not significantly modify the adhesive properties of the composition.

Among components that can be present in the aqueous suspension of nanoparticles, one can mention: mineral or organic ions, small organic molecules, proteins, physiological fluids. Notably, such components can be anti infectives, anti bacterians, preservatives, antibiotics, PEG, polymers of varied nature... Among small organic molecules, one can mention solvents like ethanol, diethylether, acetone...

In some embodiments, the nanoparticles are applied on the surface as a suspension containing a solvent, in particular an organic solvent. Said solvent may be suitable for improving suspension stability and for helping the particles to adsorb on the surface. The reason is that when the solvent evaporates (and/or penetrates a tissue or the material (e.g. a gel or a membrane or a film)) it leaves nanoparticles adsorbed onto the surface. The second role of the co-solvent that is not necessarily a good solvent of gel or tissue chains and thus it deswells the surface layer and favors gluing. Typically organic solvents include but are not limited to alcohols, diols and aprotic solvents. In a particular embodiment, the nanoparticles are deposited with a solution containing a mixture of water with an organic solvent. Typically the solution is an alcohol containing solutions or a solution containing a mixture of water and alcohol. In particular, alcohol/water mixture can be used to disperse particles containing OH groups at the surface and can thus be useful to disperse organic tightly cross-linked degradable particles.

Concentrations are adjusted to obtain suitable viscosities for application. Alternately, powders can also directly adsorb to the surface of a hydrogel.

In general, suspensions of viscosity from about 10 Pa.s or less are used, preferably lower viscosities ($10^{-3}$ Pa.s). For non-spherical particles, like particles of CNT, or CNC type, the concentration is adjusted so that the viscosity remains fairly low.

The pH of the aqueous suspension of nanoparticles can be of any value from 1 to 14 and is adapted according

to the application. pH can be adjusted to optimize adsorption, for example for anionic polyacrylic acid (PAA) macromolecules on the silica surface (Wisniewska, M. (2010), Journal of Thermal Analysis and Calorimetry, 101(2), 753-760. doi:10.1007/s10973-010-0888-4) but also to keep the stability of the nanoparticles composition. For polyelectrolyte or amphoteric gels, the pH of the nanoparticles composition is adjusted to obtain nanoparticles of charges opposed to gel's charges.

- The hydrogel:

Gels are solid, jelly-like materials based on a dilute cross-linked structure within a fluid. Gels do not flow in the steady-state. The major part of a gel is a liquid, yet they behave like solids due to their three-dimensional cross-linked network structure within the liquid. The crosslinked structure can be based on polymers of any nature or on surfactant molecules.

A hydrogel, also named a colloidal gel, is based on a network of chains, generally of the polymer type, in which water is the dispersion medium. Hydrogels can contain over 99.9% water and can be based on natural or synthetic polymers.

Hydrogels have varied properties, among which one can mention:

Hydrogels are rich in water (or solvent), yet they can be manipulated. They are conformable. Hydrogels have the capacity to create or maintain a moist environment

Some hydrogels are sensitive to external stimuli, which are also known as 'Smart Gels' or 'Intelligent Gels'. These hydrogels have the ability to sense changes of pH, temperature, electrical current, or the concentration of a molecule in their environment, like a metabolite. In reaction to such a change they can contract or swell or release their load as result of such a change. Hydrogels that are responsive to specific molecules, such as glucose or antigens, can be used as biosensors.

Hydrogels can comprise molecules dispersed in the gel, grafted on the chains which constitute the gel or solubilized in the aqueous medium. Notably culture media can be included in a gel, which can be used as a carrier for cell culture. Active principles can be included in a hydrogel which can be used for the controlled release of said actives. For example cosmetic or therapeutic active principles can be included in a hydrogel.

Hydrogels find use in numerous applications including:

- Microfluidic, wherein they can be used as valves;
- Cell culture: cell culture plaques can include hydrogel-coated wells, microbiochips can include gel coated locations for cell culture;
- Gel permeation, either classical gel permeation or microfluidic separation;
- Actuation;
- Tissue engineering: hydrogels can be used as scaffolds containing or supporting cells for tissue repairing;
- Sustained-release actives delivery, notably as cosmetic or pharmaceutical patch or dressing;
- Cooling gels for intense pulsed light or laser hair removal;
- Medical applications: biosensors, medical electrodes, contact lenses, tissue encapsulation;
- Surgery: as suturing strip, or to avoid rupture of amniotic membrane after amniocentesis;
- Food: jelly, tofu, some industrial cheeses and dairy products, surimi, are examples of mainly protein-based gels of animal or vegetal origin,
- Agrochemistry: they can be used as granules for holding soil moisture in arid areas and/or delivering nutrients to the soil.
- Art restoration and conservation: as materials for the leaning of paintings, sculptures and other work of art.

The invention is directed to a kit comprising a hydrogel that can be selected from any of this list of objects, and a composition of nanoparticles.

All kinds of hydrogels can be concerned by the invention. More specifically, the invention is concerned by gluing hydrogels of synthetic or natural origins.

Among synthetic hydrogels, one can mention:

A hydrogel of a polymer selected from: PDMA (poly(N,N-dimethyl acrylamide)., poly-NIPAM (poly(N-isopropylacrylamide)), a synthetic or extracted gel based on proteins, like gelatin, collagen, or on polysaccharides, like agarose, methylcellulose, hyaluronan, chitosan...

Such synthetic gels can comprise other molecules, like active principals (cosmetic, pharmaceutics), nutrients, proteins, cells, physiological fluids...

In addition, many human or animal tissues are based on gel or gel-like materials and will be considered as hydrogels in the context of the invention. Among natural hydrogels which are concerned by the invention, one can mention biological tissues, notably viscera, like liver, kidneys, lungs, intestines but also blood vessels, muscles, tendon,

cornea...

Favorite nanoparticles for gluing human or animal tissues are selected from: silica nanoparticles, magnetic nanoparticles (like iron oxides for medical diagnosis). However, to the difference of some prior art use of the magnetic nanoparticles, they are used as gluing agent, not for transferring heat from a laser source to their environment to promote welding between two biological tissues or between synthetic polymers.

The invention is more specifically directed to the gluing of non self adhesive hydrogels. Self adhesion can be simply tested by pressing two pieces of a gel between two fingers and observing if the gel pieces adhere or separate when one part is lifted. Among self-adhesive hydrogels known from the prior art, one can mention polymer/clay nanocomposites. However, according to the invention, the compositions of nanoparticles can be used to increase adhesion between a self-adhesive hydrogel and another article.

The hydrogel can be part of a more complex article:

For example, in some embodiments, the hydrogel is associated to a woven or non woven fabric used as biomedical prostheses and scaffolds for tissue engineering, or as cosmetic or dermatological patch to provide controlled release of biologically active molecules. They can be biodegradable or not in nature and are obtained by numerous manufactured methods including electrospinning to have small pore size, high porosity and high surface area. The association can result from adhesion of the hydrogel to the fabric or inclusion of the fabric in the hydrogel.

- The other article:

The other article which is used in the invention can be of any material to which it is desired to make a hydrogel adhere. The choice of materials for the other article is a function of the polarizability and materials on which the nanoparticles can be adsorbed. The other article can be of a material selected from: a hydrogel, a glass, a polymer, a biological tissue, a metal.

When the other article is a hydrogel, it can be identical to or different from the first hydrogel. For example, thanks to the gluing method according to the invention, one can make an assembly comprising a superposition of hydrogels of identical or similar chemical nature, presenting a gradient of a given property (concentration, pH, ionic strength). Alternately, one can make an assembly of gels of different chemical natures.

Among polymers to which a hydrogel can be glued or adhered, one can mention polycarbonate, polystyrene, polymethylmethacrylate, polypropylene, etc.

A hydrogel can be made to adhere to a glass thanks to the method disclosed here. This type of assembly is of interest in laboratory equipment like: microfluidics, biochips, gel permeation equipment...Preferably, before gluing the hydrogel, the glass is submitted to a treatment favoring the formation of reactive functions at its surface like a plasma treatment

Before gluing, the surfaces can be treated specifically by anti-microbial compounds, cationic compounds, depending on the pH and pKa of the nanoparticles composition, so that nanoparticles can be adsorbed.

A hydrogel can be made to adhere to a biological tissue, like skin or nails. For example, a patch containing a cosmetic or therapeutic active in a hydrogel can be adhered to the skin by spreading an aqueous suspension of nanoparticles to the surface of the gel before applying it to the skin. It can thus provide a continuous or delayed, or prolonged release of the active principle. An artificial nail of hydrogel nature can be adhered to the nail. Medical electrodes of hydrogel material can also be adhered to the skin by this method. A provisional or permanent prosthetic device including a hydrogel can be adhered to any tissue, like a blood vessel, and nanoparticles can create sufficient adhesion to permit suturing and/or biological tissue auto-repairing. Tissue repair hydrogels can be adhered to damaged tissue, like burnt skin.

And the same method of gluing can be used to make a hydrogel adhere to a mechanical support (polymer film, non-woven polymer network for example) when manufacturing an article like a patch or a dressing, an implant, a contact lens, a medical electrode.

Same method can be used to manufacture hydrogel composite or to reinforce hydrogels, using nanoparticle solutions as a coating to ensure good adhesion between the gel and the filler as reinforcing fibres (short fibers, continuous fibers, interwoven fibers, non-woven mats or foams, lamina reinforced composites or dispersed fillers).

- An adhesion between two parts of an organ of a gel or gel-like nature can be built thanks to nanoparticles suspension, for repairing damaged organs after an accident or during a surgical intervention. The organ can then self-repair thanks to cell colonization. The nanoparticles suspension can also be used for provisionally maintaining together two parts of an organ for suturing. Currently, damaged liver is repaired by wrapping the organ in a textile until self-repair is achieved. However, such a method presents a risk of infection due to the presence of the textile. Using a suspension of nanoparticles, notably a suspension of hydrocolloïdal silica as surgical glue presents numerous advantages: it is safe, cheap, efficient, easy to use.
- The method for gluing a hydrogel to an article:

The invention finds application in a method for adhering or gluing or creating adhesion or increasing adhesion between at least one hydrogel and at least one other article, wherein said method comprises:

a- applying a composition of nanoparticles on at least one face of the hydrogel
b- applying the face of the hydrogel bearing the nanoparticles to the article,

The method is remarkably simple: A composition of nanoparticles of the desired composition is prepared or a commercial composition is used. This composition of nanoparticles is applied on at least one face of the hydrogel. The face of the hydrogel on which the nanoparticles have been applied is applied to the article. In conformity with the skilled professional's knowledge, by applying is meant depositing on, contacting, approximating to create contact between surfaces.

According to a favorite variant, the composition is an aqueous suspension of nanoparticles.

According to another embodiment, the composition is a powder composition.

According to one favorite variant, the nanoparticles are solid nanoparticles.

Typically, the preparation of nanoparticles of the invention is applied using conventional techniques. Coating, dipping, spraying, spreading and solvent casting are possible approaches. More particularly, said applying is manual applying, applicator applying, instrument applying, manual spray applying, aerosol spray applying, syringe applying, airless tip applying, gas-assist tip applying, percutaneous applying, surface applying, topical applying, internal applying, enteral applying, parenteral applying, protective applying, catheter applying, endoscopic applying, arthroscopic applying, encapsulation scaffold applying, stent applying, wound dressing applying, vascular patch applying, vascular graft applying, image-guided applying, radiologic applying, brush applying, wrap applying, or drip applying.

According to one variant, the composition of nanoparticles is applied to the article and the face of the article on which the nanoparticles have been applied is applied to the hydrogel. According to this variant, the article is the vehicle that is used to apply the composition of nanoparticles to the hydrogel. According to this variant, applying the composition of nanoparticles to the hydrogel and contacting the hydrogel with the article are achieved in one single step.

The invention finds application in a method for adhering or gluing or creating adhesion or increasing adhesion between at least one hydrogel and at least one other article, wherein said method comprises:

a- applying a composition of nanoparticles on at least one face of the article
b- applying the face of the article bearing the nanoparticles to the hydrogel.

In some embodiments, in particular for cutaneous application, the nanoparticles can be deposited on the tissue with means typically selected from the group consisting of a patch, a dressing, an elastoplasts or a band-aid having a plurality of capsules (e.g. nanocapsules) having the ability to release the nanoparticles (e.g. in the form of powder or a solution) when they are contacted by the tissue (e.g. because of a variation of temperature, physical pressure, osmotic pressure...). Then after a while the means can be pull out, and the material or tissue can be approximated with the tissue where the nanoparticles were adsorbed.

Adhesion is achieved without heating, notably at room or body temperature to the difference of some prior art methods (WO03/026481).

For both synthetic and biological hydrogels, strong and rapid adhesion is achieved at room or body temperature by simply spreading a droplet of nanoparticle solution on the gel surface before bringing the two pieces into contact.

Alternately, the powder composition is spread at the surface by known means and the excess (non adsorbed) powder is removed.

If the composition of nanoparticles is spread in excess on the hydrogel face, the excess can be wiped away before the hydrogel treated face is contacted with the articles. Alternately, excess composition is eliminated by pressing the article and the hydrogel.

The invention finds application in a method for gluing at least one hydrogel to at least one other article, wherein said method consists essentially of:

a- applying a composition of nanoparticles on at least one face of the hydrogel
b- applying the face of the hydrogel bearing the nanoparticles to the article,
c- eliminating nanoparticles composition in excess.

Step b and step c can be achieved in any order.

The invention also relates to any of the methods as above disclosed, wherein said method comprises a preliminary step (before step a) of selecting nanoparticles capable of adsorption at the hydrogel's surface.

The invention finds application in a method comprising a step of selecting nanoparticles capable of adsorption at the other article's surface before step a.

Checking adhesion of nanoparticles to the surface of the hydrogel or the other article can for example be achieved by any of the above-disclosed methods, notably FTIR-ATR, by SEM, or by the method of thermal isotherms.

The invention finds application in a method wherein adhesion is achieved at a temperature inferior or equal to 40°C. Adhesion of the assembly can be checked by any method known to the skilled professional, examples are provided in the experimental part. Preferably, adhesion is achieved at a temperature inferior or equal to 38°C.

In the case wherein the assembly is of two synthetic materials, adhesion is preferably achieved at a temperature inferior or equal to room temperature.

In the case wherein the assembly is of a biological tissue to another biological tissue or to another material, adhesion is achieved preferably at a temperature inferior or equal to body temperature.

The optional step of drying the hydrogel can comprise complete drying of the hydrogel or incomplete drying. When the method comprises a step of drying the hydrogel between step a and step b, it can also comprise a step of hydrating the hydrogel before applying the hydrogel to the other article. When the method comprises a step of drying the hydrogel between step a and step b, the dried hydrogel can be directly applied to the other article. Notably, when said other article itself is hydrated, it can contribute to hydrate the nanoparticles layer and the hydrogel.

The above-disclosed method can comprise any of the following steps between steps a (applying a composition of nanoparticles to one face of the hydrogel) and step b:

A step of sterilizing the hydrogel,
A step of packaging the hydrogel,
A step of storing the hydrogel.

The invention finds application in a method comprising a step of applying pressure to the assembly of the hydrogel and the other article during or after step b.

The invention finds application in a cosmetic method for gluing a hydrogel to the skin.

For gluing biological tissues together or for gluing a biological tissue to another article, it is preferred to use an aqueous suspension of nanoparticles. This choice provides improved cicatrization as compared to nanoparticles in powder.

Just after the hydrogel and the other article have been contacted the hydrogel can be repositioned and behaves like a pressure sensitive adhesive. After some time has elapsed, the adhesion is stronger and the hydrogel can no longer be peeled.

The invention finds application in a method for gluing at least one hydrogel to at least one other article, wherein said method does not need exposure to laser radiation.

The invention finds application in a method for gluing at least one hydrogel to at least one other article, wherein said method does not comprise exposure to laser radiation.

The invention finds application in a method for gluing at least one hydrogel to at least one other article, wherein said method does not comprise heating the nanoparticles.

The quantity of nanoparticles deposited at the surface of the hydrogel is advantageously from 0.1 mg/$m^2$ to 10 g/$m^2$. Depending on the size of the nanoparticles, the coverage of the surface is to be adjusted. These values can be from 1 mg/$m^2$, preferably for small particles, and up to 0.2 g/$m^2$, preferably for large particles. For large particles (typically of the order of 300 nm) the coverage is large, of the order of 4 g / $m^2$. For particles of smaller size (diameter of about 2 nm) rates coverage is preferably of the order of 10 mg / $m^2$.

When a droplet of a nanoparticle solution is spread on one surface, the quantity of nanoparticles «deposited» per surface area is much larger then 10g/$m^2$ (as illustrated in the examples). However, two objects that are glued are pressed into contact and the solution is basically squeezed outside the junction. Hence, essentially, only the particles that were adsorbed (attached) onto the objects' surfaces are present at the interface and bring adhesion. Hence, after bringing into contact (pressing objects) the concentration of the particles should be (basically) within the limits of from 0.1 mg/$m^2$ to 10g/$m^2$. In the presence of blood flow or when gluing a porous gel or a porous tissue, the excess of particles is further washed away. It thus seems that deposited is meant as adsorbed, attached, located or just present.

Preferably, it is believed that optimum adhesion is obtained for a dense monolayer on the nanoparticles surface. The density of coverage can be evaluated on the assembly by ATR-FTIR or by SEM.

In some embodiments, the volume of nanoparticles that is deposited at the surface ranges from 0.01 to 5 μl per $mm^2$.

By "layer of nanoparticles" is meant a monolayer or several layers. A layer, or layers, of nanoparticles can be continuous or non continuous.

In some embodiments, the nanoparticles are deposited and adsorbed on the two surfaces that shall be adhered (i.e. the two synthetic materials, the two tissue surfaces, the tissue surface and the material surface). However, in a preferred embodiment only one surface is adsorbed with the nanoparticles. For example, when a material shall adhere to a tissue, it is preferable to absorb the nanoparticles on the material surface rather than on the tissue surface.

In some embodiments, it may be desirable to get only one layer of nanoparticles. In some embodiments, the step b of applying the face of the hydrogel to the article comprises a manual approximating, a mechanical approximating, a suture approximating, a staple approximating, a synthetic mesh approximating, a biologic mesh approximating, a transverse approximating, a longitudinal approximating, an end-to-end approximating, or an overlapping approximating.

It is known to the skilled professional that to create or increase adhesion, the two materials making the assembly must be kept in contact for a certain amount of time. It is known to the skilled professional that to create or increase adhesion one can apply a more or less important pressure to the assembly.

In some embodiments, in or after step b, the two surfaces are applied or contacted to each other for a time ranging from 5s to 2min, preferably from 10s to 1 min, more preferably from 20s to 50s.

In some embodiments, in or after step b, the two contacted surfaces are submitted to a pressure for a time ranging from 5s to 2min, preferably from 10s to 1 min, more preferably from 20s to 50s.

In some embodiments, the nanoparticles are just absorbed on the surface of the hydrogel before being applied to the other article.

Typically, the physician that would like to adhere a material on a tissue prepares the material as above described by adsorbing the nanoparticles to the surface of the material. Then he approximates the material and the tissue for a time sufficient for allowing the surfaces of the material and the tissue to adhere to each other.

In some embodiments, the nanoparticles are previously adsorbed on the surface of the hydrogel. Accordingly, the invention can be carried out by use of ready-to-use synthetic hydrogels that can be prepared in an industrial manner and then be stocked in a proper manner.

Once the user would like to make the hydrogel adhere to the other article, he just has to release the material and proceed to step b of the method.

Such an embodiment includes several variants:

When the hydrogel comprising adsorbed nanoparticles has been stored in a humid state, it can be used without any preparation. For example, it is not necessary to hydrates the material before applying it. The material, such as hydrogel can thus be applied directly to a tissue or to another article, and will automatically adhere to it.

When the hydrogel comprising adsorbed nanoparticles has been stored in a dry state, it can be also used without any preparation if the other article itself is humid. In that case, it is not necessary to hydrate the material before applying it. For example the dried hydrogel will naturally swell in contact of the biological fluids present in the implantation site (e.g. blood, lymph, exudates...), or in contact with the water present in another synthetic hydrogel.

When the hydrogel comprising adsorbed nanoparticles has been stored in a dry state, it can be necessary to hydrate the material before applying it.

In step a, the hydrogel is typically prepared as described above. For example, the hydrogel may be immerged in an aqueous suspension of nanoparticles for a sufficient time for allowing the nanoparticles to adsorb to the surface of the hydrogel. Alternatively, an amount of nanoparticles is deposited on the surface of the hydrogel with a brush that was previously dipped in an aqueous suspension of nanoparticles. The aqueous suspension of nanoparticles may also be sprayed on the surface of the hydrogel. Then the hydrogel can be dried, optionally lyophilized, sterilized, packaged and properly stocked for a subsequent use. In some embodiments, a powder of nanoparticles is dispersed (e.g. by spraying) on the surface of the hydrogel and the excess is then washed. Then the hydrogel is optionally lyophilized, sterilized, packaged and properly stocked for a subsequently use.

As illustrated in the experimental part, the inventors achieved strongly bonding together pieces of hydrogels, which have either the same or different chemical nature or rigidity.

The method has the advantage of being very simple. It does not request additional steps like pH adjustments, drying of the nanoparticles composition after application, nanoparticles activation or excitation, for example through laser irradiation...

Surprisingly, the step of contacting the hydrogel already treated with the composition, like for example the suspension, of nanoparticles with the other article, can be achieved under water. A good adhesion of the two elements is obtained according to this variant. For gels that are not at swelling equilibrium when the assembly is immersed in water, both the gels and bond layer swell.

To glue fully swollen gels, according to a favorite variant, the surface layer is just slightly dried before applying the nanoparticles composition. Alternately, fully swollen gel can be glued thanks to an appropriate choice of nanoparticles (size and affinity).

The invention finds application in a method for adhering or gluing or creating adhesion or increasing adhesion between at least one hydrogel and at least one other article, wherein said method consists essentially of:

a- applying a composition of nanoparticles on at least one face of the hydrogel
b- applying the face of the hydrogel bearing the nanoparticles to the article.

By "consists essentially of' is meant that, if other steps are present in the method, they do not significantly

interfere with adhesion. For example, a variant of this method is a method wherein the composition of nanoparticles is applied both on the hydrogel and on the other article: Finally, the composition of nanoparticles finds itself at the interface between the hydrogel and the nanoparticles and adhesion is not significantly different as compared to the case when the composition is applied solely on the hydrogel. On the contrary, the method wherein the adhesion is achieved thanks to another compulsory step, like nanoparticles heating by laser excitation, is a method that comprises one other essential step for adhesion to be achieved.

The invention finds application in a method for adhering or gluing or creating adhesion or increasing adhesion between at least one hydrogel and at least one other article, wherein said method consists essentially of:

a- applying a composition of nanoparticles on at least one face of the article
b- applying the face of the article bearing the nanoparticles to the hydrogel.

When the article or the hydrogel is selected from biological tissues *in vivo,* the method is a surgical method or a therapeutic method. It can be a method for repairing a damaged organ, or for adhering an implant, a prosthesis, a patch or a dressing to a biological tissue. Alternately, it can be a cosmetic or dermatological method, comprising the application of a cosmetic or dermatological patch to the skin or to the nails.

As compared to the prior art, the method disclosed has the advantage that nanoparticles compositions can be used for adhering two biological tissues or a biological tissue and another article, without having to prepare a fibrin glue in a separate step.

As disclosed in the experimental part, to illustrate the promise of the method for biological tissues, pieces of calf liver were glued to obtain, in a dozen of seconds, manipulable assemblies.

As exemplified herein after the methods disclosed may find very various medical applications. In particular the methods disclosed provide the following advantages. First of all, the methods disclosed may be used directly in vivo even in presence of body fluids such as blood, lymph, exudates, urine, bile, intestine contents... Accordingly the methods disclosed can be applied in tissue that are normally perfused or can be applied to tissues that are leaking (e.g. blood). In particular the inventors surprisingly demonstrate that nanoparticles can be adsorbed on the tissue surfaces in a sufficient manner for adhering even if a part of them is flowed by the presence of the body fluid, in particular blood. Secondly the methods disclosed offer the advantage to maintain the physical, chemical and biological integrities of the tissue where the nanoparticles are adsorbed. In particular, as demonstrated by the inventors, no physical barrier is created (as generally observed with glues made of cyanoacrylate) that will prevent the tissue diffusion, e.g. the circulation of the biological molecules, cells (e.g. immune cells) or fluids between the adhering tissues or between the material (e.g. hydrogel) and the tissue. In particular, the physical properties of the tissue are maintained in particular the elasticity of the tissue. Thirdly the methods of the present invention are very easy to settle and may be performed very quickly in very different conditions (temperature, presence of body fluids, organ or tissues in motion (e.g. a beating heart)...). The adhesion offer by the method disclosed may be a permanent adhesion or a temporary adhesion. For example, one skilled in the art can imagine that the methods disclosed may be performed during a surgery procedure so as to prevent in urgent manner a leaking of blood vessels till the surgeon stabilizes the haemostatic assembly with sutures, meshes or staples.

Accordingly in some embodiments, the methods disclosed are particularly suitable for sealing a defect between a first and second tissue in the subject.

In some embodiments, the methods disclosed are particularly suitable for providing a barrier to, for example, microbial contamination, infection, chemical or drug exposure, inflammation, or metastasis.

In some embodiments, the methods disclosed are particularly suitable for stabilizing the physical orientation of a first tissue surface with respect to a second tissue surface.

In some embodiments, the methods disclosed are particularly suitable for reinforcing the integrity of a first and second tissue surface achieved by, for example, sutures, staples, mechanical fixators, or mesh.

In some embodiments, the method disclosed of the present invention is particularly suitable providing control of bleeding.

In some embodiments, the methods disclosed are particularly suitable for delivery of drugs including, for example, drugs to control bleeding, treat infection or malignancy, or promote tissue regeneration.

According to one embodiment, none of the article and the hydrogel is selected from biological tissues *in vivo.*

According to this embodiment the article or the hydrogel can independently be selected from or include *ex vivo* or *in vitro* isolated or cultured biological tissues. The article or the hydrogel can independently also be of a synthetic material or a natural material of non human, and non animal origin.

- <u>Assembly of a hydrogel and an article:</u>

The invention gives access to an assembly of at least one hydrogel and at least one other article, wherein the

interface between the at least one hydrogel and the at least one other article is or consists essentially of a layer of nanoparticles. In the assembly disclosed, in case the at least one hydrogel or the at least one other article is a biological tissue, it is an isolated tissue or a cultured tissue. By "consists essentially of' is meant that, if other elements are present at the interface, they do not significantly interfere with adhesion.

By layer of nanoparticles, is meant a monolayer or multi-layers of nanoparticles.

In the assembly, the concentration of nanoparticles at the interface is preferably from 0,1 $mg/m^2$ to 10 $g/m^2$, advantageously from 1 $mg/m^2$, preferably for small particles, and up to 0.2 $g/m^2$, preferably for large particles

Such an assembly can in a non limitative manner be selected from: a microfluidic equipment, a biochip, a gel permeation equipment, an actuation gel assembly, a food gel assembly, a cosmetic or pharmaceutical patch or dressing, a biosensor, a medical electrode, a contact lens, an implant, a prosthesis.

The invention is remarkable in that the adhesion between the hydrogel and the article is resistant to humidity. Glued assemblies can resist immersion and swelling in water.

Further, the assembly is remarkable in that it holds thanks to adhesion, not welding or any other mechanism.

According to a favorite variant of the assembly, the nanoparticles are selected from materials that cannot be heated, notably that cannot be heated by a laser excitation method. For example, they may be selected from organic nanoparticles and clays, silicates, alumina, silica, kaolin, grafted carbon nanotubes, grafted cellulose nanocrystals, hydroxyapatite.

The preparation of an assembly disclosed is illustrated in figure 1. Figure 1a: two hydrogels (1) and (2) are placed opposite with a layer of nanoparticles (3) in between. The two hydrogels are pressed and both are in contact with the nanoparticles (3). In the adhesive layer, nanoparticles (3) act as connectors between gel pieces (1) and (2) and gel-chains (1.1) (2.1) bridge different particles (Fig. 1b).

- Kits:

The invention also gives access to a kit for gluing a hydrogel to an article, wherein said kit comprises at least a hydrogel and at least a composition, preferably an aqueous suspension, of nanoparticles.

According to this variant, when the hydrogel is a biological tissue, it is a cultured or isolated tissue.

Preferably, according to this variant, the hydrogel is a synthetic hydrogel.

Advantageously, the nanoparticles suspension used in the method for gluing a hydrogel to another article presents the characteristics above disclosed.

Alternately, the nanoparticles composition is a nanoparticles powder composition.

The nanoparticles used in the kit are capable of adsorption at the hydrogel's surface.

In some embodiments, the kit comprises means for distributing the nanoparticles on the surface of the hydrogel and/or other article (dripper, spray, vaccum, pipette or sealed pipette, patches, dressing, elastoplasts band-aid or brush for example).

Said kit can comprise two or more compartments for separately conditioning the hydrogel and the composition of nanoparticles and for permitting an optimized use thereof. For example the kit can comprise a collection of hydrogels packaged in independent compartments and a flask comprising the suspension of nanoparticles with an appropriate distribution means (dripper, spray or brush for example). Alternately, it can comprise one hydrogel and the appropriate quantity of nanoparticles suspension for gluing said gel. In said kit, the hydrogel can be part of a more complex article. One example is a kit wherein the hydrogel is part of a cosmetic or dermatological patch, or a dressing. Such an article generally comprises a scaffold of another material in addition to the hydrogel.

Accordingly, a further embodiment of a disclosed kit relates to a hydrogel as above described wherein an amount of nanoparticles is adsorbed on at least one surface of the material. Said hydrogel may be stored in a dry or humid state and is properly stocked for a subsequent use. Typically, the hydrogel was previously sterilized and packaged.

According to a variant, the invention gives access to a surgical kit, wherein it comprises at least one composition of nanoparticles, preferably consisting essentially of a suspension of nanoparticles, and at least one article chosen from: a suturing needle, a suturing strip, suturing staples, a prosthesis, an implant. The two parts are conditioned in sterile containers. They are complementary, since the suspension of nanoparticles can be used to glue in a provisional manner two parts of an organ, or an organ and a medical device (a prosthesis for example). Said gluing makes suturing by a known method easier. Said surgical kit can comprise any other article of use, like a hydrogel for example. The nanoparticles used in the kit are advantageously capable of adsorption to biological tissues.

In some embodiments, the surgical kit comprises means for distributing the nanoparticles on the surface of the hydrogel and/or other article (dripper, spray, vaccum, pipette or sealed pipette, patches, dressing, elastoplasts band-aid or brush for example).

The invention has been described with reference to preferred embodiments. However, many variations are possible within the scope of the invention.

- Experimental part:

I- Methods summary

- Nanoparticles suspensions:

Silica Ludox® TM-50, HS-40, and SM-30 water solutions with, respectively, concentration of 52, 40, and 30 wt% at pH 9, 9.5 and 10, SiO2/Na20 ratio of 200-250, 89-101, and 45-56, radii of about 15, 9, and 5 nm, were purchased from Aldrich and used as received.

Silica particles AL-30 were synthesized using Stöber et al. method (W. Stöber W., A. Fink, E. Bohn, Controlled growth of monodisperse silica spheres in the micron size range. Journal of colloid and interface science 26, 62-69 (1968)). In particular 600 mL of absolute ethanol and 36 mL of ammonium hydroxide solution (35 wt.% in water) were added to a round bottom flask and stirred for 5 min. 18 mL of TEOS were then quickly poured and the resulting solution was stirred overnight at room temperature. Silica particles were retrieved by centrifugation (8500 rpm, 45 min) and washed with absolute ethanol and followed by four cycles of centrifugation-dispersion. Silica particles were eventually air dried over 3 hours at 100°C. Particles characterization was performed using dynamic light scattering (DLS) and transmission electron microscopy (TEM). The particles hydrodynamic radius (DLS) was about 60 nm and the polydispersity index was about 4%. The radius determined from TEM images analysis was about 50 nm. Larger AL-300 particles were prepared using the same method. In this case, 90 mL of absolute ethanol, 90 mL of deionized water and 14 mL of ammonium hydroxide solution (35 wt.% in water) were added to a round bottom flask and stirred for 5 min. 56 mL of TEOS were then quickly poured and the resulting solution was stirred overnight at room temperature. The particles hydrodynamic radius determined by DLS was about 200 nm and the polydispersity index was about 4%. Unless otherwise stated AL30 and AL300 nanoparticles were used dissolved in deionized water at 30 wt% (pH=8.5).

The synthesis of the silica nanoparticles stabilized by end-grafted poly(oxyethylene) chains (PEGylated silica nanoparticles) JL-100, was carried out in two steps using Toon et al. method (Toon T.-J., Kim J.S., Kim B.G., Yu K.N., Cho M.-H., Lee J.-K., Multifunctional nanoparticles possessing a "magnetic motor effect" for drug or gene delivery, Angew. Chem. Int. Ed., 2005, 44, 1068-1071). In the first step, the PEG-silane precursor was obtained by reacting poly(ethyleneglycol) tosylate (2g, $M_n$=2000 g/mol, Sigma-Aldrich) with aminopropyl triethoxysilane (0.18g, Sigma-Aldrich) in 20mL of dimethylformamide (DMF).

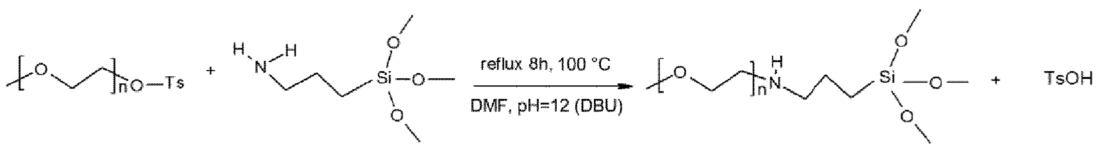


pH was adjusted to 12 by adding 1,8-diazabicyclo[5.4.0]undec-7-ene (DBU, Sigma-Aldrich) and the reaction mixture was refluxed at 100°C for 8 hrs. After DMF evaporation and re-dissolution in chloroform, the product was precipitated and washed with n-hexane and dried under vacuum. In the second step, silica nanoparticles were synthesized according to the Stöber method (W. Stöber W., A. Fink, E. Bohn, Controlled growth of monodisperse silica spheres in the micron size range. Journal of colloid and interface science 26, 62-69 (1968)). 9.0 mL tetraethylorthosilicate (TEOS, Sigma-Aldrich) was added into a mixture of 300 mL ethanol and 18 mL 35% ammonia under strong stirring at room temperature. Thus synthesized silica nanoparticles had the hydrodynamic radius as measured by DLS of about 67$\pm$3 nm. After 24 hours, the PEG-silane precursor (1g in 2 mL methanol) was added and the dispersion was heated to 60°C for 8 hrs. PEGylated silica nanoparticles were washed and centrifuged several times with ethanol and water and dried at 80°C overnight. The hydrodynamic radius of JL-100 PEGylated particles was determined by DLS to be about 74$\pm$3 nm. JL-10 nanoparticles were used dissolved in deionized water at 20 wt% (pH=8.5).

Multi-wall carbon nanotubes (CNT) were supplied by Arkema (Graphistrength® C100) and purified with sulphuric acid. Thymine-grafted CNT particles (CNT-Thy) were synthesized via method of Prevoteau, A., Soulié-Ziakovic, C. & Leibler, L. Universally Dispersible Carbon Nanotubes. J. Am. Chem. Soc. 134, 19961-19964 (2012).

Cellulose nanocrystals (CNC1) bearing sulfate and hydroxyl groups have been prepared via method of Bondeson, D., Mathew, A. & Oksman, K. Optimization of the isolation of nanocrystals from microcrystalline cellulose by acid hydrolysis. Cellulose 13, 171-180 (2006). Cellulose nanocrystals CNC2 were prepared using the same method, but replacing sulfuric by chlorhydric acid. The suspensions diluted to desired concentration (0.5 and 3% wt% for CNTs and CNCs, respectively) were sonicated for 30min just before use.

- Hydrogels:

PDMA and PDMA nanocomposite gels were prepared via method of Carlsson, L., Rose, S., Hourdet, D. & Marcellan, A. (Nano-hybrid self-crosslinked PDMA/silica hydrogels. Soft Matter 6, 3619-3631 (2010)). Polyacrylamide hydrogels were prepared by *in -situ* free radical polymerization of acrylamide using thermal dissociation of potassium persulfate (KPS) as initiator, at 80°C. *N,N'*-methylenebisacrylamide (MBA) was used as cross-linker and MBA/DMA ratio was 0.1, 0.5, 1 and 1.5 mol.%, for samples S0.1, S0.5, S1.0 and S1.5, respectively and MBA/AAm was 0.1 mol.% for A0.1 gel. At the preparation state, gels matrix hydration was fixed at 87.7 wt%. To avoid network defects that lead to a very weak self-adhesion of gels it is important to conduct synthesis under nitrogen conditions. For PDMA M0.1 gels MBA/DMA ratio was 0.1. In contrast to PDMA S0.1 gels, M0.1 were synthesized under nitrogen conditions, but not in a glove box. As shown in the tables below when compared with S0.1 gels, M0.1 gels exhibit a slightly lower elastic Young modulus and higher equilibrium swelling. This suggests a presence of pending chains in the network structure (J. Bastide, C.Picot, S. Candau The influence of pendent chains on the thermodynamic and viscoelastic properties of swollen networks, J.Polym.Sci. :Physics Ed. 17, 1441-1456 (1979)). M0.1 gels are weakly self-adhesive.

Gelatin (Technical 1, VVR) gels were prepared by cooling, from 50°C to RT, 23 wt% aqueous solutions.

Table 1a and 1b summarize gel properties. Here, swelling degree *Q* is defined as the ratio of hydrated gel volume to the dry polymer volume. *E* denotes the tensile modulus at the preparation state, $Q_0$ or at the swelling equilibrium conditions, $Q_e$, respectively.

Table 1A: Properties of PDMA and gelatin gels

| **Sample** | **Preparation state** | | | | | | | |
|---|---|---|---|---|---|---|---|---|
| | **water (g)** | **MA (g)** | **AAm (g)** | **MBA (mg)** | **Silica vol. fraction** | **Gelatin (g)** | **Q** | **E (kPa)** |
| **S0.1** | **10.62** | **1.485** | | **2.3** | | | **8.5** | **10 ± 2** |
| **NC** | **10.62** | **1.485** | | **2.3** | **0.21** | | **8.5** | **93 ± 8** |
| **S0.5** | **10.69** | **1.485** | | **11.6** | | | **8.5** | **26 ± 1** |
| **S1.0** | **10.77** | **1.485** | | **23.0** | | | **8.5** | **45 ± 2** |
| **S1.5** | **10.94** | **1.485** | | **46.2** | | | **8.5** | **60 ± 4** |
| **M0.1** | **10.62** | **1.485** | | **2.5** | | | **8.5** | **9.5±1** |
| **A0.1** | **7.62** | | **1.065** | **2.3** | | | **9** | **11 ± 1** |
| **Gelatin** | **11.1** | | | | | **3.31** | **5.3** | **30 ± 2** |

Table 1B: Properties of PDMA and gelatin gels

| **Sample** | **Swelling equilibrium conditions** | | |
|---|---|---|---|
| | **Q** | **E (kPa)** | **Swelling ratio $Q_e/Q_0$** |
| **S0.1** | **41 ± 1** | **4 ± 1** | **5.1** |
| **N** | **29 ± 4** | **7 ± 1** | **3.4** |
| **S0.5** | **23 ± 3** | **- -** | **2.7** |
| **S1.0** | **17 ± 2** | **- -** | **2.0** |
| **S1.5** | **15 ± 2** | **- -** | **1.7** |
| **M0.1** | **52±5** | | **5.8** |
| **A0.1** | | | |
| **Gelatin** | **29±2** | | **4.9** |

In particular, we present elastic PDMA gels properties for a large range of crosslink densities and elastic moduli For the synthesis of these PDMA gels, 0.041g of potassium persulfate (KPS) and 22.5 μL of *N*,*N*,*N'*,*N'*-tetramethylethylenediamine TEMED were introduced as redox initiators. For the preparation of polyacrylamide A0.1 gels: two aqueous solutions were prepared: KPS at 4.5wt% and MBA at 1.2 wt%. First, MBA solution and AAm were dissolved at 25°C in

water. The KPS solution was added and the homogeneous solution was purged with nitrogen during 15 minutes under magnetic stirring. The mixture was finally transferred, under nitrogen atmosphere, into laboratory-made moulds previously sealed and put under nitrogen atmosphere. The sealed moulds were placed in an oven at 80°C for 24 hours. Gelatin gels were prepared by dissolving the gelatin powder in deionized water under stirring at 50°C during two hours. The gelatin concentration was 23 wt.%. The mixture was then introduced in moulds and left at room temperature during 30 minutes. Moulds were stored at 6°C during two days prior testing and left at room temperature for 1 h before testing.

- Testing methods:

Lap joint geometry: Displacement was measured by a video extensometer that followed two markers (white dots), which were placed at 5 mm distance from the edge of the lap joint. The total length of assembled ribbons was 40 mm. *w* denotes the width and *h* the thickness of gel ribbons. *l* is the overlap length.

Scanning electron micrographs were obtained using a Field Emission SEM (Hitachi SU-70).

Lap-shear and mechanical tests were performed on an Instron 5565 tensile testing machine equipped with a 10N load cell and an optical extensometer, at a speed of 150 mm/min. For mechanical tests PDMA gel samples were cut into rectangular ribbon shape of 50 mm x 5 mm x 2 mm. Single lap-shear geometry was used with a joint of 10 mm length by 5 mm width. Extensometer markers were placed 5 mm from the edge of the lap joint. The initial distance between markers was about 20 mm for gels at preparation conditions $Q_0$ and correspondingly larger for gels at $Q_e$. The total length of the assembled ribbons was 40 mm. In order to avoid systematic failure in the vicinity of the clamps, gelatin samples had a dog-bone shape following the ISO4661-1 standard with the reduced section of samples of 25 mm x 4 mm x 2 mm. Lap joint dimensions were 10mm x 4mm.

When interfacial failure occurs by peeling, from the measured adhesive failure force *F* the adhesion energy can be calculated (Kendall, K. Molecular Adhesion and Its Applications. (Plenum Publishing Corporation, 2001; Kendall, K. Cracking of Short Lap Joints. The Journal of Adhesion 7, 137-140 (1975)), $G_{adh}=3(F/w)^2/(2Eh)$, where *w* and *h* denote, respectively, the width and thickness of the ribbon, and E is the tensile modulus.

Fracture tests were performed using the classical single edge notch geometry using the Instron machine. For that purpose, a cut was made in the centre of the rectangular samples using a blade. Each notch was measured by optical microscopy in order to determine its exact length (*c*), approximately 1 mm. The same procedure as the one used for tensile tests was performed: the strain rate was fixed to be 0.06 $s^{-1}$, the force and the displacement data were recorded. In the case of a single edge notched specimen, Rivlin, Thomas and Greensmith determined the fracture energy as:

$$G_{IC} = 2KW(\lambda_c)c$$

with *W* the strain energy density, $\lambda c$ the extension ratio at break and c the initial length of the crack. (Rivlin, R.S., Large Elastic Deformations of Isotropic Materials .4. Further Developments of the General Theory. Philosophical Transactions of the Royal Society of London Series a-Mathematical and Physical Sciences, 1948. 241(835): p. 379-397 ; Greensmith, H.W., Rupture of rubber. X. The change in stored energy on making a small cut in a test piece held in simple extension. Journal of Applied Polymer Science, 1963. 7(3): p. 993-1002.)

Thermal isotherms are determined by the method of Hourdet, D. and L. Petit (2010). Macromolecular Symposia. C. S. Patrickios. Weinheim, Wiley-V C H Verlag Gmbh. 291-292: 144-158. Different series of mixtures of nanoparticle and polymer chains (of the same chemical nature as the gel) were prepared by introducing increasing amounts of polymer (N-acrylamide, PNIPA, PEO) into the nanoparticle suspension Ludox TM-50. The samples are then stored for several days to provide sufficient time to the polymer chains to be adsorbed onto the nanoparticle surface and to equilibrate. The nanoparticles are then settled down by centrifugation and the supernatant is recovered for titration. The total concentration of free polymer chains in the supernatant (Cp) was determined by titration using GPC (Gel Permeation Chromatography) or using a total organic carbon (TOC) analyser or other methods of titrations. The amount of adsorbed polymer on nanoparticle surfaces ($\Gamma$ in mg/$m^2$) is estimated and is plotted against the equilibrium polymer concentration.

The isotherms illustrated in Hourdet et al. immediately give evidence that both PDMA and PNIPA interact more energetically than PEO with silica surfaces. In the case of PDMA and PNIPA, a strong adsorption regime is observed at low coverage of the particles, with a sharp increase of the adsorbed amount of polymer for $\Gamma/\Gamma_{max}<0.5$ ($\Gamma<0.5-0.7$ mg/$m^2$). This regime is followed by a weaker interaction domain ($\Gamma/\Gamma_{max}>0.5$) and ends up at the plateau value, which is approximately the same for the two polymers: $\Gamma_{max} \cong 1$ mg/$m^2$. The same holds for PEO chains but with a smaller extent as the plateau value is reached at $\Gamma_{max} \cong 0.6$ mg/$m^2$. Applying the Langmuir isotherm model in the high coverage regime ($\Gamma/\Gamma_{max}>0.5$, Figure 1), the adsorption equilibrium constant (K) can be estimated and is seen to increase from PEO to PNIPA and then to PDMA: K=3, 6 and 30 L.$g^{-1}$, respectively.

From these results, we can anticipate that silica nanoparticle solutions enable the gluing of PDMA and PNIPAM

gels. But silica nanoparticles can also be modified to adsorb onto poly(acrylamide) gels and to provide adhesive properties to the poly(acrylamide) gel (Pefferkorn, E., A. Carroy, et al. (1985). Macromolecules 18(11): 2252-2258.) or pH can be adjusted to optimize adsorption, for example for anionic polyacrylic acid (PAA) macromolecules on the silica surface (Wisniewska, M. (2010). Temperature effect on adsorption properties of silica-polyacrylic acid interface. Journal of Thermal Analysis and Calorimetry, 101(2), 753-760. doi:10.1007/s10973-010-0888-4)

Fourier transform infrared spectroscopy (FTIR) coupled with Attenuated total reflectance (ATR)

Ludox TM-50 shows good adhesive properties with S0.1 gels at preparation state, $Q_0$. On the other hand, very weak bonding properties are obtained when the substrate is at its maximum swelling equilibrium, $Q_e$.

ATR-FTIR was used in order to compare the adsorption properties of Ludox TM-50 on S0.1 at $Q_0$ and on S0.1 at $Q_e$. For that purpose, two samples of S0.1 gels containing the same polymer weight were used, for one at $Q_0$ and for the other one at $Q_e$. Both samples were immersed in Ludox TM-50 solution for 30 seconds then soaked in a large volume of deionized water during 3 days, exchanging the solvent every 12 hours. Prior to ATR-FTIR analysis the swollen S0.1 samples were dried (at 80°C for 2 days).

Fourier transform infrared spectroscopy was carried out using a Bruker TENSOR TM27 spectrometer fitted with a diamond ATR accessory. Figure 13 shows the ATR-FTIR spectra of the surfaces of S0.1 gel submitted to this preparation process. Presence of silica nanoparticle is tracked by the 1100 $cm^{-1}$ band and reveals that adsorption is weaker when the S0.1 gel is at its maximum swelling equilibrium. Adhesive properties confirm this observation. Silica band assignment is given from Parida et al. (Parida SK, Dash S, Patel S, and Mishra BK. Advances in Colloid and Interface Science 2006;121(1-3):77-110.) and references wherein: 800 $cm^{-1}$ $\delta$(OH) silanol ; 975 $cm^{-1}$ $\nu$(Si-OH) ; 1100 $cm^{-1}$ $\nu_{as}$(Si-O-Si) 1630 $cm^{-1}$ $\delta$(O-H) molecular water. Gel spectra were normalized using the 1400 $cm^{-1}$ band assigned to $\delta_S(CH_3)$ of poly(dimethylacrylamide) hydrogel (Sekine Y and Ikeda-Fukazawa T. Journal of Chemical Physics 2009; 130(3).) and references wherein v is the stretching and $\delta$ is the bending mode.

Scanning Electron Microscopy (SEM) in combination with Energy Dispersive X-ray (EDX) spectroscopy.

EDX is an analytical technique used for the elemental analysis or chemical characterization of a sample. The first micrometers of the surface are probed. The sample preparation procedure is the same as for the ATR-FTIR test. Figure 14a and 14b compare the morphology and element analysis of the surfaces of S0.1 gel: S0.1 at Qo (a) and S0.1 at $Q_e$ (b) prepared according to the above disclosed protocol. SEM micrographs and EDX results confirms that silica adsorption is weaker for S0.1 at Qe.

II- Experiments

- Example 1:

Water soluble polymers from substituted acrylamides such as poly(dimethylacrylamide) (PDMA) or poly(n-iso propyl acrylamide), readily adsorb to silica nanoparticles (Hourdet, D. & Petit, L., Macromol. Symp. 291-292, 144-158 (2010)), whereas poly(acrylamide) chains do not adsorb onto silica (Griot, O. & Kitchener, J. A. Role of surface silanol groups in the flocculation of silica suspensions by polyacrylamide. Part 1. Chemistry of the adsorption process. Trans. Faraday Soc. 61, 1026 (1965)). To demonstrate the concept of nanoparticles as adhesives and test the importance of gel chain adsorption onto particles, we tested hydrogels, S0.1, made of poly(dimethylacrylamide) (PDMA) and A0.1, made of poly(acrylamide) (PAAm). Both gels had the same cross-linking density, 0.1 mole% and contained 88 wt% of water (Table 1). Both PDMA S0.1 and PAAm A0.1 gels did not adhere to themselves. When a 15 $\mu$L drop of TM-50 silica suspension was spread on PDMA gel surface (w=5mm and 1=10mm) and another PDMA piece was pressed to form a lap-junction, a strong adhesion was observed after few seconds of contact. In contrast, for poly(acrylamide) gels, even when we pressed hardly and for a very long time, we could not make lap junctions that held under their own weigh and thus confirmed that the lack of gel chains adsorption onto nanoparticles prevents gluing.

Please note that silica surface can be modified to enable the adsorbtion of poly(acrylamide) chains as described by Pefferkorn, E., A. Carroy, et al. (1985). Macromolecules 18(11): 2252-2258.). For example, the authors modified the silica surface by firstly making it hydrophilic by soaking for 24 h in hot hydrochloric acid medium and then by treating with aluminum chloride in chloroform in order to replace some silanols (SiOH) by aluminum chloride groups, which by hydrolysis of the latter are converted to aluminol (AlOH).

To test the strength of adhesion brought by TM50 silica nanoparticle solutions we performed shear lap joint tests on PDMA S0.1 gels and found that for all lap joints with large overlap length *l* (between 4 and 20 mm) the failure systematically occurred outside the bonding junction, showing that the joint was stronger than the gels themselves (Fig. 9a, 2a, 4). Lap joints with small overlap length or which are made of narrow and thick gel ribbons rotate under tensile load and interfacial failure by peeling occurs. From measured adhesive failure force *F* (Fig. 9a) we can evaluate the adhesion energy (Kendall, K. Molecular Adhesion and Its Applications. (Plenum Publishing Corporation, 2001; Kendall, K. Cracking of Short Lap Joints. The Journal of Adhesion 7, 137-140 (1975)), $G_{adh}=3(F/w)^2/(2Eh)$, where *w* and *h* denote, respectively, the width and thickness of the ribbon, and *E* is the tensile modulus. We found $G_{adh}$ to be $6.6\pm1.6$ J/$m^2$ and

6.2±1.4 J/$m^2$, respectively, for short (*l*=2mm, *w*=5mm, *h*=2mm) and narrow and thick (*l*=5mm, *w*=2mm, *h*=5mm) joints (Fig. 2b).

- Example 2:

To probe how the size of silica particles affects the adhesion, failure force *F* was measured in a lap shear tensile test with geometry (w=5mm, h=2mm, l=5mm) that offers a good compromise between adhesive joint weakness and measurement precision (Fig. 9b, Fig. 6). In this geometry adhesive failure by peeling was observed for junctions glued with smaller particles (SM-30 and HS-40 with radii 5 and 9 nm, respectively) and bulk fracture outside the junction for bigger particles (TM-50 and AL-30 with radii 15 and 50 nm, respectively). Using AL-30 particles led to bulk failure even when the joints were very short, narrow and thick. To induce peeling making cuts at interface was necessary. Thus, it seems that adhesion, strong in all cases, increased when particle size was increased, although it should be noted that changing the size of particles implies some variations of the surface chemistry as well.

- Example 3:

Particle surface chemistry can be harnessed to bring (i.e. promote) adhesion by improving suspension stability or by promoting specific interactions such as hydrogen bonding that strengthen the particle adsorption to gel surface. Thus grafting thymine to carbon nanotubes brought (i.e. significantly increased) adhesion. Similarly, cellulose nanocrystals CNC1 bearing sulfate groups yield adhesion strength comparable with that obtained with nanosilica whereas CNC2 particles with hydroxyl groups only were useless as a glue (i.e. gluing properties are not as good as those obtained with silica) for S0.1 gels (Fig. 9b).

- Example 4:

Gluing strength also depends on gel properties. In tightly cross-linked gels, strands are more constrained and there is a higher entropy penalty for adsorption (Johner, A. & Joanny, J.-F. Adsorption of polymeric brushes: Bridging. J. Chem.Phys. 96, 6257 (1992)). In addition, energy dissipation mechanisms discussed above are less efficient for short strands *N*. Adhesion strength is therefore expected to be weaker for more rigid gels. Figure 9b shows that indeed adhesion energy *Gadh* decreased by a factor of 3 when the crosslinking degree of bonded gels was increased by a factor of 10 corresponding to increase of tensile modulus by a factor of 4.5. At the same time, more rigid gels dissipate less energy during fracture and are more brittle as evidenced by single edge notched tensile test results (Fig. 9b). Hence, in practice, even for rigid gels, gluing by nanoparticles is sufficiently strong to allow designing of joints that withstand tensile stresses without adhesive failure (Fig. 3a, 3b, 3c). It is also possible to glue strongly gels with very different rigidities (Fig. 7a).

- Example 5:

Gluing by nanoparticle solutions is not limited to hydrogels that are in the as synthesized state. Dehydration of gels before gluing is expected to increase adhesive strength, whereas gel swelling should have the opposite effect. Indeed, in a more swollen state strands are more stretched and adsorb less readily. Choosing particles with right size and affinity to gels becomes important. We used AL-30 silica solution to glue swelled S0.1 gel ribbons and measured *Gadh* as a function of swelling degree Q (Fig. 4e). Although lower by a factor of 2.4 when compared with gels at *Q*=16, the adhesion energy at the maximum (equilibrium) swelling $Q_e$=41 (97,6 v/v% of water) was as high as 1.6±0.6 J/$m^2$.

- Example 6:

The design principle assures that adhesion remains when the joint is immersed in excess solvent and swells since particles once adsorbed stay strongly anchored to the gel surfaces. Indeed, the probability of total desorption of gel chains is exponentially small (P.G. de Gennes, Polymers at an interface; a simplified view, Advances in Colloid and Interface Science 27, 189-209 (1987)). To confirm strong anchoring of adsorbed nanoparticles, we spread a droplet of TM-50 silica solution on the gel surface and washed the surface in pure water several times. Scanning electron microscopy showed that nanoparticles densely cover the surface, even after washing (Fig. 5b). We then glued S01 hydrogels having swelling degree of $Q_0$=8.5 with TM-50 nanoparticles and immersed lap joint in water to reach the maximum, equilibrium swelling of $Q_e$=41. The junction withstood five-fold volume increase and adhesion energy measured in lap-shear test was 1.8±0.5 J/$m^2$, i.e. 3.5 times lower than in preparation state (Fig. 5d, 4a). In the fully swollen state adhesion strength is weaker because detaching adsorbed chains is easier as adsorbed chains are already under higher swelling tension. Moreover, once detached the swollen strand is less prone to adsorb and strand-strand exchange dissipation processes

are hindered.

Example 7:

Strong irreversible anchoring of once adsorbed particles brings an attractive possibility of self-repairing and/or re-positioning adhesive joints. Figure 5d shows that a joint, which was peeled, can recover its initial strength when ribbons are brought into contact and pressed with fingers for few seconds without any need to re-apply the glue (Fig. 8, 5d).

- Example 8:

Particle solutions offer simple method of gluing gels of different chemical nature provided particle surface chemistry is properly adjusted to allow adsorption on both gels. For example, using TM-50 silica solution a robust assembly of S0.1 and gelatin was achieved (Fig. 7b). Gelatin/PDMA S0.1 junction glued with TM-50 silica was immersed in water for one week to reach maximum equilibrium swelling did not break. Swelling ratios of gelatin and S0.1 gels are comparable ($Q_e/Q_0$=5) although their moduli are very different. Because of the stiffness mismatch the joined gels deform and curve under swelling, but the interface holds.

In many applications such as actuation, gluing gels of different rather than identical chemical nature presents advantages, including the possibility of assembling gels with different rigidity, but similar equilibrium swelling. Such assemblies can withstand equilibrium (maximum) swelling in excess water (Fig 12). In contrast, swelling of a glued assembly of chemically identical gels with mismatched swelling capacities can lead to high, heterogeneous osmotic stresses near the interface and produce slow interfacial failure. Glued at their preparation state by TM-50 solution, both PDMA S0.1 and PDMA S1.5 gels had initially the same size (diameter of about 10 mm). After 5 hours of swelling in deionised water, highly cross-linked PDMA S1.5 gel is less swollen than PDMA S0.1 gel. Gel S0.1 shows a fivefold over-swelling when immersed in water, whereas a more tightly cross-linked gel S1.5 over-swells by a factor of 1.7 only. As a result, interfacial stresses induced by heterogeneous over-swelling exceed considerably shear stresses applied in mechanical lap-shear tests. Hence, for S0.1/S1.5 assemblies, interfacial failure was observed during immersion and over-swelling in water. Still adhesion joint held for quite a long time and the de-bonding was slow.

- Example 9A:

Soft biological tissues although incomparably more complex, mechanically and osmotically, resemble gels in many respects. To test gluing potentialities of nanoparticle solutions we cut from calf liver two ribbons 45x18x3 $mm^3$. Cut pieces do not adhere to each other and cannot be glued by water at pH 9. We spread 60$\mu$L of silica TM-50 solution on cut surface (without any pre-treatment or special drying) to make a lap joint with overlap length $l$=20 mm. After being pressed for 30 seconds with a finger (contact pressure of about $10^5$ Pa), the lap joint held strongly and could be manipulated with ease.

Normalized force-displacement curves are illustrated in figure 10. Lap shear adhesion tests for two different calf livers yield adhesion energy $Gadh \approx 25 \pm 5$ J/$m^2$ and $Gadh \approx 6 \pm 1.6$ J/$m^2$. The moduli of two livers are respectively 15.0$\pm$1.7 kPa and 12$\pm$1.5 kPa.

Example 9B:

Nanoparticles suspensions provide a simple method for gluing biological tissues with gels. For example, Ludox TM-50 was used to glue calf liver to gelatin gel. We cut from calf liver one ribbon of 45x25x3 $mm^3$ and a gelatin sample was cut into rectangular shape of 15x20x3 $mm^3$. The gelatin sample was soaked in TM-50 silica solution for 30 seconds then brought into contact and pressed to the calf liver surface for 30 seconds. The obtained gelatin/liver assembly can sustain handling and immersion in deionized water for several hours.

Example 10: Study of PDMA S0.1 gels gluing and influence of the volume of nanoparticles suspensions deposited

Silica solution Ludox TM-50 is used. Different volumes of silica are deposited on a junction of length = 15 mm, w = 5 mm in width and h = 2 mm thickness. A pressure of 10 kPa is exerted for 30 seconds. The joint is tested in the geometry of overlay covering (lap shear tensile test).

We obtain the results illustrated in Figure 15, which show that, whatever the volume of Ludox TM-50 deposited, cohesive failure outside the junction is observed.

Example 11:

Droplets of AL300 silica nanoparticles solutions were deposited on a M0.1 PDMA gel surface having dimensions of 10mm x 5mm. The droplet is spread on the gel surface. We varied the volume of the droplets from 5 to 60 microleters. After time *t* the gel is immersed in deionized water for 1 minute and gel surface is delicately washed. The water in the bath becomes turbid indicating that some nanoparticles are not adsorbed (attached) to the surface of the gel and are removed during washing. The gel is then removed and immersed in deionized water and the surface on which nanoparticle solution was spread is washed for the second time. The gel surface is studied by ATR-FTIR and intensity of a peak characteristic to SiO2 is observed confirming that some silica nanoparticles are irreversibly attached (adsorbed) to the gel surface. After drying the samples in IR spectra bands that can be attributed to PDMA and silica are observed and intensity at 1100 $cm^{-1}$ assigned to $\nu_{as}$(Si-O-Si)) normalized using intensity at 1400 $cm^{-1}$ band assigned to $\delta_S(CH_3)$ of poly(dimethylacrylamide) hydrogel gives a measure of quantity of adsorbed silica nanoparticles concentration. Kinetics studies of silica adsorption as a function of time *t* show that for all droplet volumes after few minutes the SiO2 peak intensity does not evolve and remains constant indicating that the adsorbed concentration essentially reaches the maximum value. Within experimental accuracy this maximum normalized intensity is the same independently of the volume of the droplet spread (Figure 16). This result indicates that the quantity of nanoparticles that are located and attached (adsorbed) onto the gel basically is constant although the quantity of the particles that are present in the spread solution increased by a factor 12. These results together with those of Example 10, which indicate that adhesion brought by nanoparticle solution is strong, independently of the volume of the solution that was spread to form a junction, seem to confirm the hypothesis that nanoparticles form a layer strongly adsorbed to the gel surface and that this layer located at and attached to the interface promotes adhesion. We performed the same adsorption study experiments for AL30 silica and obtained similar results that for AL300 particles. However, the observed normalized peak intensity for the smaller AL30 nanoparticles was roughly 2 times lower than the intensity recorded for larger AL300 particles. This observation seems to be roughly consistent with and support further a notion of nanoparticles monolayer attached to gel surface.

Example 12

To demonstrate the notion that adsorbed solid nanoparticles can act as an adhesive we performed shear lap joint tests on PDMA M0.1 gels. 5 microliters of AL30 nanoparticles is spread on 20mm x 5mm surface at one end of 35mm x 5mm x 2mm gel ribbon. After 1 minute, the gel surface is washed by a procedure described in Example 11 and dried for about 30 seconds at ambient conditions. The end of the ribbon onto which nanoparticles were adsorbed is put into contact with a PDMA M0.1 gel ribbon to make a lap joint with dimensions $l$=20mm, $w$=5mm ($h$=2mm). The tensile test is performed and adhesion stronger than in the absence of nanoparticles is observed (Figure 16). The failure occurs by peeling in the adhesive junction and therefore for such large junctions the adhesion energy can be estimated using the formula: $G_{adh}= (F/w)^2/(4Eh)$ (K. Kendall, J. Phys. D: Applied Physics, 8, 512 (1975)). We find $G_{adh} \approx 1.4 \pm 0.5$ J/$m^2$ in the presence of adsorbed nanoparticles at the junction and $G_{adh} \approx 0.6 \pm 0.1$ J/$m^2$ for control experiment in the absence of nanoparticles.

In order to show that nanoparticles act as connectors and are attached to both gels we studied the peeled gels surfaces by ATR-FTIR and found that indeed after adhesive failure (peeling) the adsorbed particles are present on both surfaces: the surface of a gel onto which they were spread and on the surface of the gel which was put into contact with the first gel.

Hence Examples 10-12 seem to confirm that solid nanoparticles that adsorb to gel can act as a gluing agent and bring adhesion.

Example 13

The small particles suspended in solutions have a strong tendency to attract when in close contact and form aggregates that sediment. Steric stabilization consists in adsorbing or grafting polymer chains in order to prevent the particles to get close in the range of attractive forces. Indeed, in a good solvent grafted or adsorbed polymer layers attached to the particle surfaces have a tendency to repel and do not interpenetrate. Unfortunately, the same entropic repulsion mechanism that helps to stabilize the particles can hinder the particle adsorption to a polymer gel. The present example illustrates this phenomenon. We study adsorption onto PDMA M0.1 gels of aqueous solutions (20 w/w%) of PEGolated silica particles (JL-100) stabilized by end-grafted poly(oxyethylene) chains. The method is described in Example 11 and results are compared with adsorption of AL30 silica particles that are stabilized only by electrostatic repulsions. The normalized IR intensity at 1100 $cm^{-1}$ is, respectively, of about 4.2 for JL-100 and of about 23.6 for AL-30 particles indicating, indeed, that grafting PEG chains hinders adsorption. We then carried lap joint adhesion tests and observed that the efficiency of JL-100 was lower than that of AL30 nanoparticles (Figure 18). This example illustrates

that for the best adhesive performance it is important to optimize particle adsorption onto surfaces of objects to be glued. It also indicates that nanoparticles suspensions that are stabilized by attaching polymers to their surfaces adsorb less readily to gels and in consequence may be less efficient as adhesives.

Conclusion:

The results suggest that nanoparticles solutions bring a way to simply assemble synthetic and biological hydrogels as well as biological tissues without affecting substantially the rigidity or permeability of the assembly. Powerful methods exist to tune and control surface chemistry of inorganic particles and organic solid particles to achieve optimal particle adsorption and bonding. The possibility to self-repair and repositionable adhesive joints is an additional boon. Given the importance of wet adhesion in biomedicine and biotechnology as well as in more traditional coating and material technologies, our results open interesting avenues to develop new applications by simply assembling all kinds of chemically and mechanically mismatched tissues and/or gels.

## Claims

1. A composition of inorganic nanoparticles, for its surgical use as gluing agent or adhesive, for adhering one first biological tissue and one second biological tissue, or for adhering one biological tissue and at least one other article, wherein the composition of nanoparticles is selected from a powder and an aqueous suspension, or aqueous dispersion or aqueous solution, of nanoparticles, wherein the composition of nanoparticles contains no more than 20% by weight of other gluing agent as compared to the weight of the dry matter of the composition.

2. A composition according to claim 1, wherein the nanoparticles represent from 20 to 100% by weight of the weight of the dry matter of the composition.

3. A composition according to claim 1 or claim 2, wherein the nanoparticles are selected from particles from 5 to 300 nm in size.

4. A composition according to any of claim 1 to 3, wherein the nanoparticles are selected from: clays, silicates, alumina, silica, kaolin, carbonaceous nanoparticles, grafted carbon nanotubes, hydroxyapatite, magnetic nanoparticles, metal oxides, noble metals, quantum dots.

5. A composition according to any of claim 1 to 4, for its surgical use for repairing damaged organs or for provisionally maintaining together two parts of an organ for suturing.

6. A composition according to any of claim 1 to 4, wherein the other article is a medical device.

7. A composition according to claim 6, wherein the other article is an implant, a prosthesis, a patch, a dressing, biosensors of hydrogel material, medical electrodes of hydrogel material.

8. Use of a composition of inorganic nanoparticles as gluing agent or adhesive between at least one hydrogel and at least one other article, wherein in case the hydrogel or the other article is a biological tissue, it is an isolated tissue or a cultured tissue, wherein the composition of nanoparticles is selected from a powder and an aqueous suspension, or aqueous dispersion or aqueous solution, of nanoparticles, wherein the composition of nanoparticles contains no more than 20% by weight of other gluing agent as compared to the weight of the dry matter of the composition.

9. Cosmetic, non-therapeutic, non-surgical, use of a composition of inorganic nanoparticles as gluing agent or adhesive between at least one hydrogel and the skin or the nails, wherein the composition of nanoparticles is selected from a powder and an aqueous suspension, or aqueous dispersion or aqueous solution, of nanoparticles, wherein the composition of nanoparticles contains no more than 20% by weight of other gluing agent as compared to the weight of the dry matter of the composition.

10. The use according to claim 8 or claim 9, wherein the nanoparticles represent from 20 to 100% by weight of the weight of the dry matter of the composition.

11. A use according to any of claim 8 to 10, wherein the nanoparticles are selected from:
clays, silicates, alumina, silica, kaolin, carbonaceous nanoparticles, grafted carbon nanotubes, hydroxyapatite, magnetic nanoparticles, metal oxides, noble metals, quantum dots.

**12.** Use according to any of claims 8 to 11, wherein the other article is of a material selected from: a hydrogel, a glass, a polymer, a biological tissue.

**13.** Use according to any of claims 8 to 12, wherein the hydrogel is not self adhesive.

**14.** Use according to any of claims 8 to 13, wherein the hydrogel is of a material selected from: poly(acrylamide derivatives), PDMA, poly-NIPAM, a synthetic or extracted gel based on proteins, a synthetic or extracted gel based on polysaccharides, poly(ethylene glycol hydrogel) (PEG), poly(vinyl alcohol) hydrogels, block copolymers of PEG and hydrophobic polyesters, a biological tissue.

**Patentansprüche**

**1.** Zusammensetzung aus anorganischen Nanopartikeln zu deren chirurgischer Verwendung als Verklebungsmittel oder Klebstoff, um ein erstes biologisches Gewebe und ein zweites biologisches Gewebe zu verkleben, oder um ein biologisches Gewebe und mindestens einen anderen Artikel zu verkleben, wobei die Zusammensetzung der Nanopartikel ausgewählt ist aus einem Pulver und einer wässrigen Suspension oder wässrigen Dispersion oder wässrigen Lösung von Nanopartikeln, wobei die Zusammensetzung von Nanopartikeln nicht mehr als 20 Gew.% anderes Verklebungsmittel enthält, verglichen mit dem Gewicht des Trockenmaterials der Zusammensetzung.

**2.** Zusammensetzung nach Anspruch 1, wobei die Nanopartikel 20 bis 100 Gew.% des Gewichts des Trockenmaterials der Zusammensetzung repräsentieren.

**3.** Zusammensetzung nach Anspruch 1 oder Anspruch 2, wobei die Nanopartikel ausgewählt sind aus Partikeln mit einer Größe von 5 bis 300 nm.

**4.** Zusammensetzung nach einem der Ansprüche 1 bis 3, wobei die Nanopartikel ausgewählt sind aus: Tonen, Silikaten, Aluminiumoxid, Siliciumdioxid, Kaolin, kohlenstoffhaltigen Nanopartikeln, gepfropften Kohlenstoffnanoröhrchen, Hydroxyapatit, magnetischen Nanopartikeln, Metalloxiden, Edelmetallen, Quantenpunkten.

**5.** Zusammensetzung nach einem der Ansprüche 1 bis 4 zur chirurgischen Verwendung zur Reparatur geschädigter Organe oder zum provisorischen Zusammenhalten von zwei Teilen eines Organs zum Vernähen.

**6.** Zusammensetzung nach einem der Ansprüche 1 bis 4, wobei der andere Artikel eine medizinische Vorrichtung ist.

**7.** Zusammensetzung nach Anspruch 6, wobei der andere Artikel ein Implantat, eine Prothese, ein Pflaster, eine Wundauflage, Biosensoren aus Hydrogelmaterial, medizinische Elektroden aus Hydrogelmaterial sind.

**8.** Verwendung einer Zusammensetzung von anorganischen Nanopartikeln als Verklebungsmittel oder Klebstoff zwischen mindestens einem Hydrogel und mindestens einem anderen Artikel, wobei, falls das Hydrogel oder der andere Artikel ein biologisches Gewebe ist, es ein isoliertes Gewebe oder ein kultiviertes Gewebe ist, wobei die Zusammensetzung der Nanopartikel ausgewählt ist aus einem Pulver und einer wässrigen Suspension oder wässrigen Dispersion oder wässrigen Lösung von Nanopartikeln, wobei die Zusammensetzung der Nanopartikel nicht mehr als 20 Gew.% anderes Verklebungsmittel enthält, verglichen mit dem Gewicht des Trockenmaterials der Zusammensetzung.

**9.** Kosmetische, nicht-therapeutische, nicht-chirurgische Verwendung einer Zusammensetzung von anorganischen Nanopartikeln als Verklebungsmittel oder Klebstoff zwischen mindestens einem Hydrogel und der Haut oder den Nägeln, wobei die Zusammensetzung der Nanopartikel ausgewählt ist aus einem Pulver und einer wässrigen Suspension oder wässrigen Dispersion oder wässrigen Lösung von Nanopartikeln, wobei die Zusammensetzung der Nanopartikel nicht mehr als 20 Gew.% anderes Verklebungsmittel enthält, verglichen mit dem Gewicht des Trockenmaterials der Zusammensetzung.

**10.** Verwendung nach Anspruch 8 oder Anspruch 9, wobei die Nanopartikel 20 bis 100 Gew.% des Gewichts des Trockenmaterials der Zusammensetzung repräsentieren.

**11.** Verwendung nach einem der Ansprüche 8 bis 10, wobei die Nanopartikel ausgewählt sind aus: Tonen, Silikaten, Aluminiumoxid, Siliciumdioxid, Kaolin, kohlenstoffhaltigen Nanopartikeln, gepfropften Kohlenstoffnanoröhrchen, Hydroxyapatit, magnetischen Nanopartikeln, Metalloxiden, Edelmetallen, Quantenpunkten.

**12.** Verwendung nach einem der Ansprüche 8 bis 11, wobei der andere Artikel ein Material ausgewählt aus einem Hydrogel, einem Glas, einem Polymer, einem biologischen Gewebe ist.

**13.** Verwendung nach einem der Ansprüche 8 bis 12, wobei das Hydrogel nicht selbstklebend ist.

**14.** Verwendung nach einem der Ansprüche 8 bis 13, wobei das Hydrogel ein Material ausgewählt aus Poly(acrylamidderivaten), PDMA, Poly-NIPAM, einem synthetischen oder extrahierten Gel auf Basis von Proteinen, einem synthetischen oder extrahierten Gel basierend auf Polysacchariden, Poly(ethylenglykolhydrogel) (PEG), Poly(vinylalkohol)-Hydrogelen, Blockcopolymeren von PEG und hydrophoben Polyestern, einem biologischen Gewebe ist.

## Revendications

**1.** Composition de nanoparticules inorganiques, pour son utilisation chirurgicale comme agent de collage ou adhésif, pour faire adhérer un premier tissu biologique et un deuxième tissu biologique, ou pour faire adhérer un tissu biologique et au moins un autre article, dans laquelle la composition de nanoparticules est choisie parmi une poudre et une suspension aqueuse, ou dispersion aqueuse ou solution aqueuse, de nanoparticules, dans laquelle la composition de nanoparticules ne contient pas plus de 20% en poids d'autre agent de collage par rapport au poids de matière sèche de la composition.

**2.** Composition selon la revendication 1, dans laquelle les nanoparticules représentent de 20 à 100% en poids du poids de la matière sèche de la composition.

**3.** Composition selon la revendication 1 ou la revendication 2, dans laquelle les nanoparticules sont choisies parmi les particules de taille allant de 5 à 300 nm.

**4.** Composition selon l'une quelconque des revendications 1 à 3, dans laquelle les nanoparticules sont choisies parmi: les argiles, les silicates, l'alumine, la silice, le kaolin, les nanoparticules carbonées, les nanotubes de carbone greffés, l'hydroxyapatite, les nanoparticules magnétiques, les oxydes métalliques, les métaux nobles, les points quantiques.

**5.** Composition selon l'une quelconque des revendications 1 à 4, pour son utilisation chirurgicale pour réparer des organes endommagés ou pour maintenir provisoirement ensemble deux parties d'un organe à suturer.

**6.** Composition selon l'une quelconque des revendications 1 à 4, dans laquelle l'autre article est un dispositif médical.

**7.** Composition selon la revendication 6, dans laquelle l'autre article est un implant, une prothèse, un patch, un pansement, des biocapteurs en matériau hydrogel, des électrodes médicales en matériau hydrogel.

**8.** Utilisation d'une composition de nanoparticules inorganiques comme agent de collage ou adhésif entre au moins un hydrogel et au moins un autre article, étant entendu que dans le cas où l'hydrogel ou l'autre article est un tissu biologique, il s'agit d'un tissu isolé ou d'un tissu cultivé, dans lequel la composition de nanoparticules est choisie parmi une poudre et une suspension aqueuse, ou une dispersion aqueuse ou une solution aqueuse, de nanoparticules, dans laquelle la composition de nanoparticules ne contient pas plus de 20% en poids d'un autre agent de collage par rapport au poids de la matière sèche de la composition.

**9.** Utilisation cosmétique, non thérapeutique, non chirurgicale d'une composition de nanoparticules inorganiques comme agent de collage ou adhésif entre au moins un hydrogel et la peau ou les ongles, la composition de nanoparticules étant choisie parmi une poudre et une suspension aqueuse, ou dispersion aqueuse ou solution aqueuse, de nanoparticules, dans laquelle la composition de nanoparticules ne contient pas plus de 20% en poids d'un autre agent de collage par rapport au poids de matière sèche de la composition.

**10.** Utilisation selon la revendication 8 ou la revendication 9, dans laquelle les nanoparticules représentent de 20 à 100% en poids du poids de la matière sèche de la composition.

**11.** Utilisation selon l'une quelconque des revendications 8 à 10, dans laquelle les nanoparticules sont choisies parmi: les argiles, les silicates, l'alumine, la silice, le kaolin, les nanoparticules carbonées, les nanotubes de carbone greffés, l'hydroxyapatite, les nanoparticules magnétiques, les oxydes métalliques, les métaux nobles, les points quantiques.

**12.** Utilisation selon l'une quelconque des revendications 8 à 11, dans laquelle l'autre article est en un matériau choisi parmi: un hydrogel, un verre, un polymère, un tissu biologique.

**13.** Utilisation selon l'une quelconque des revendications 8 à 12, dans laquelle l'hydrogel n'est pas auto-adhésif.

**14.** Utilisation selon l'une quelconque des revendications 8 à 13, dans laquelle l'hydrogel est en un matériau choisi parmi: les dérivés de polyacrylamide, le PDMA, le poly-NIPAM, un gel synthétique ou extrait à base de protéines, un gel synthétique ou extrait à base de polysaccharides, de l'hydrogel de polyéthylène glycol (PEG), des hydrogels d'alcool polyvinylique, des copolymères séquencés de PEG et de polyesters hydrophobes, un tissu biologique.

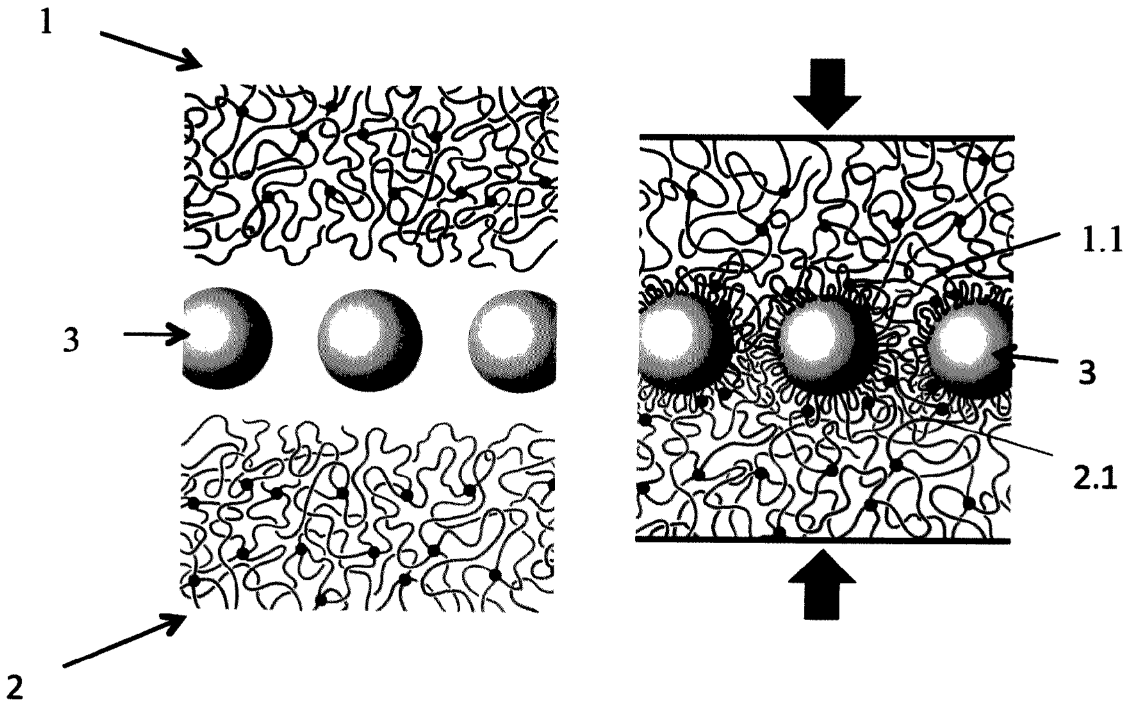


1a

1b

FIGURE 1

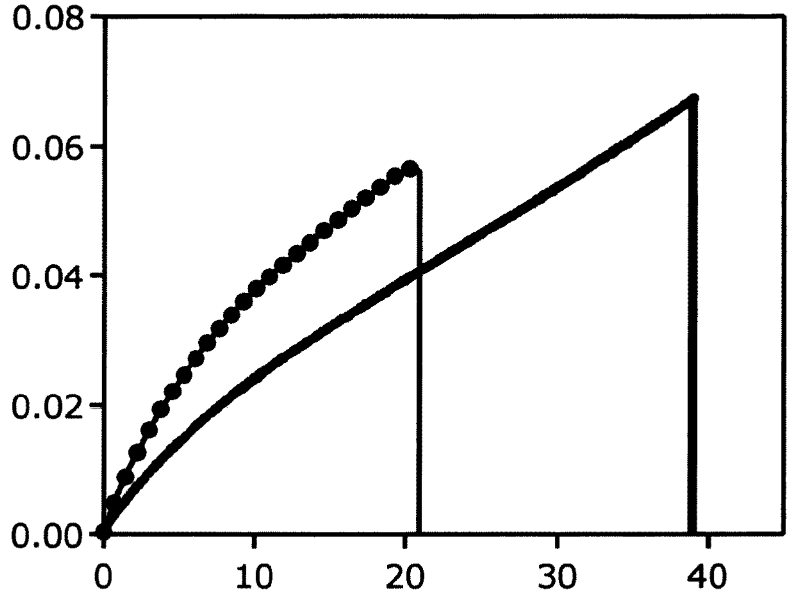


FIGURE 2A

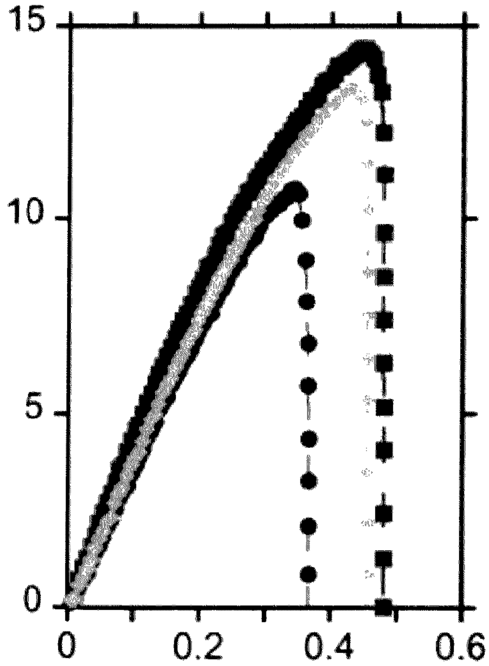


FIGURE 2B

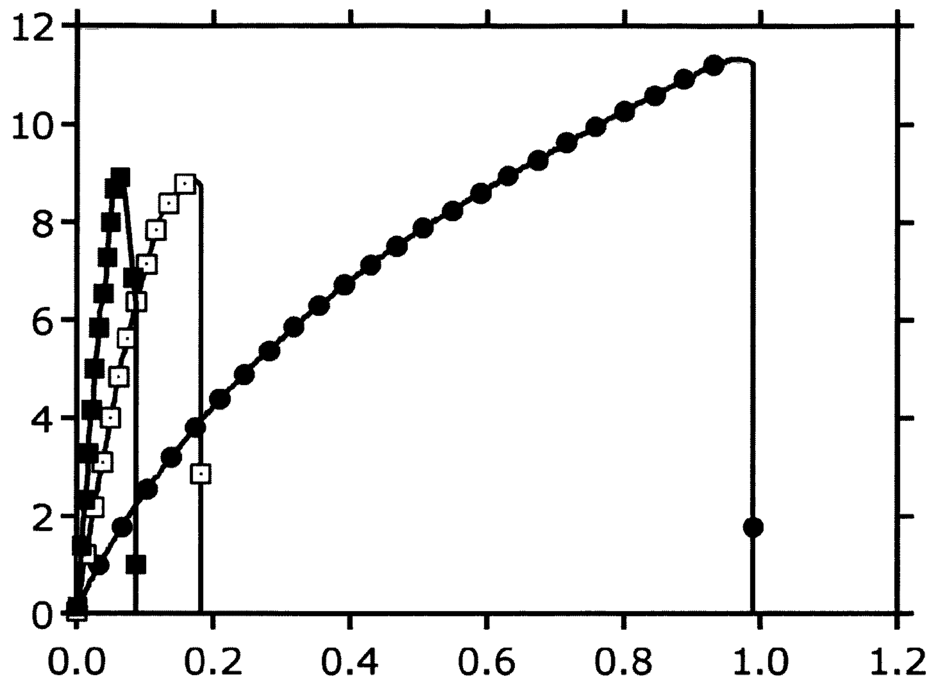


Figure 3a

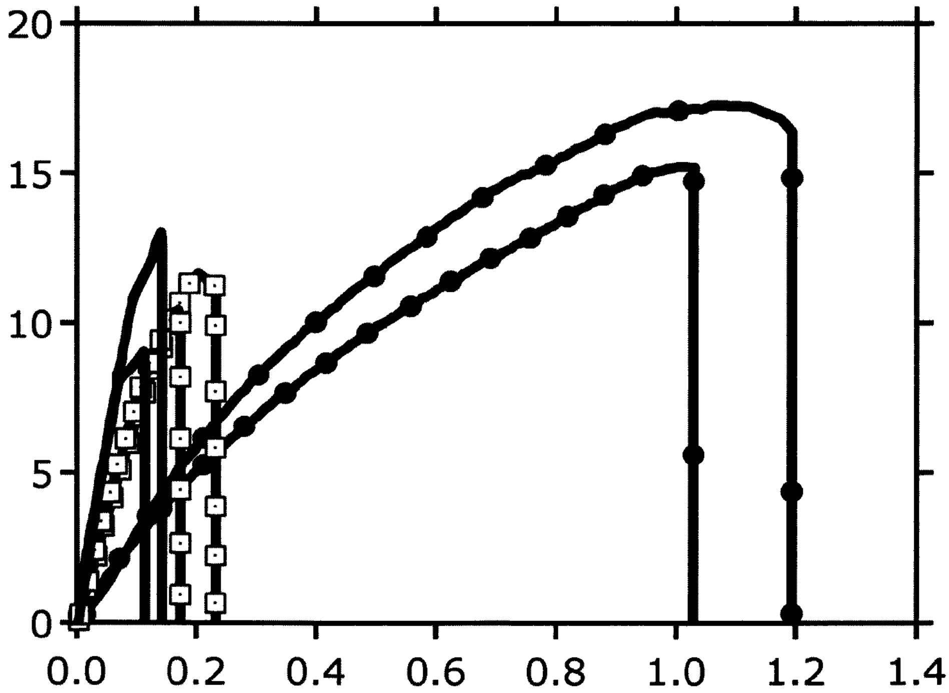


FIGURE 3B

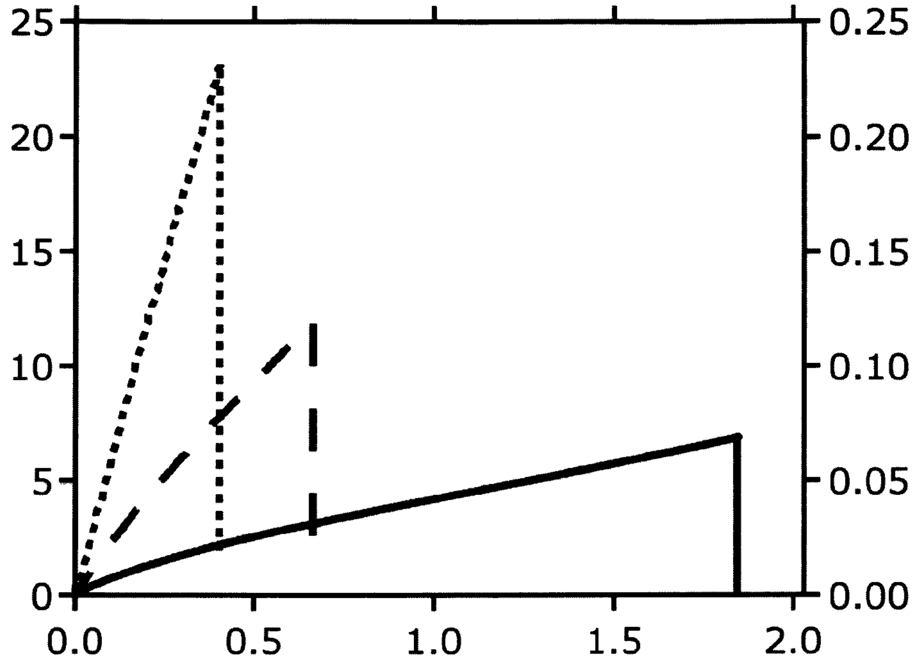


FIGURE 3C

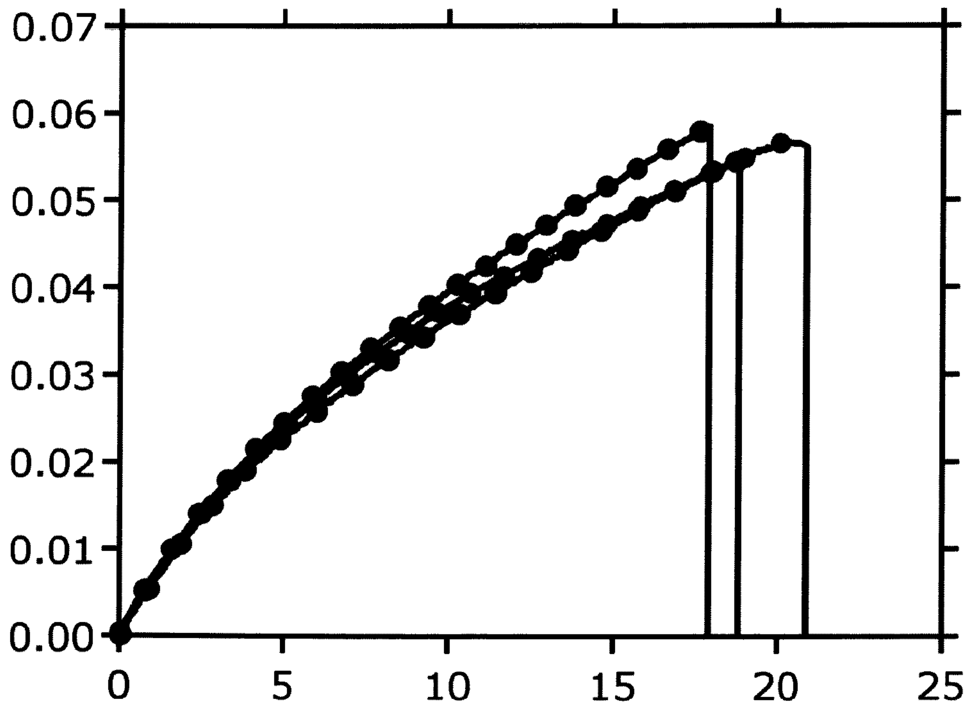


FIGURE 4

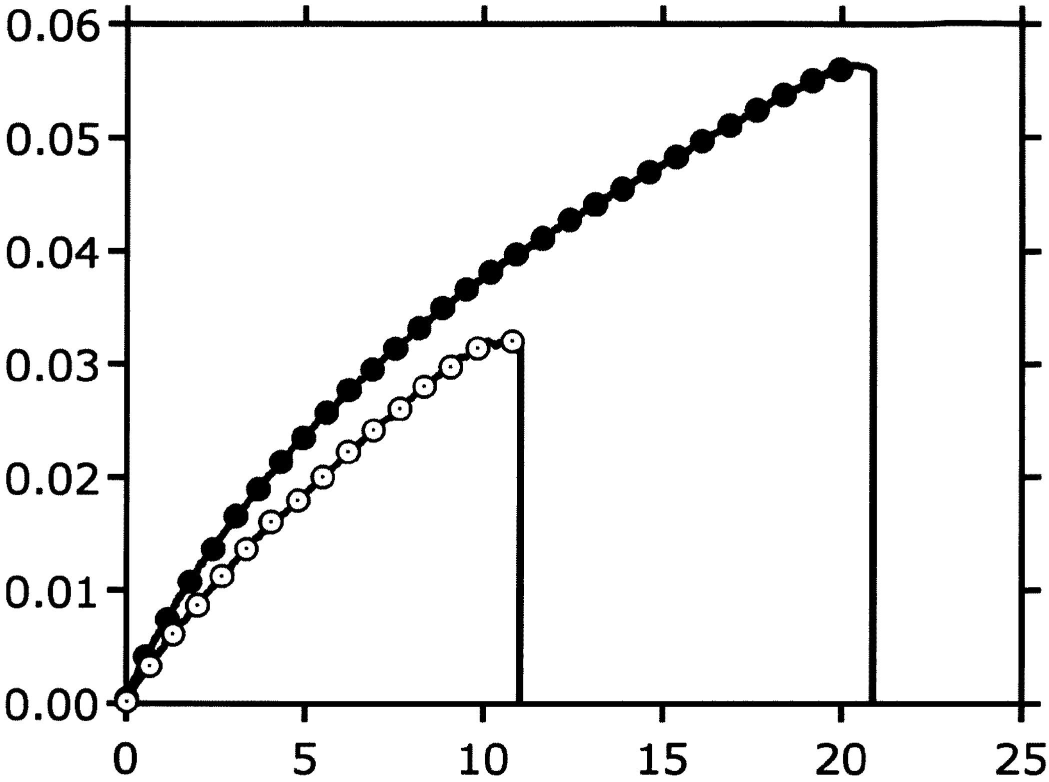


FIGURE 5A

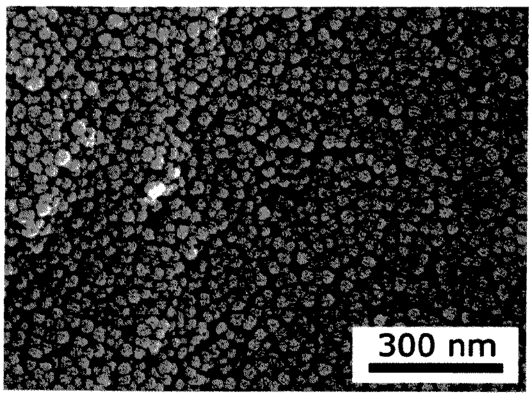


FIGURE 5B

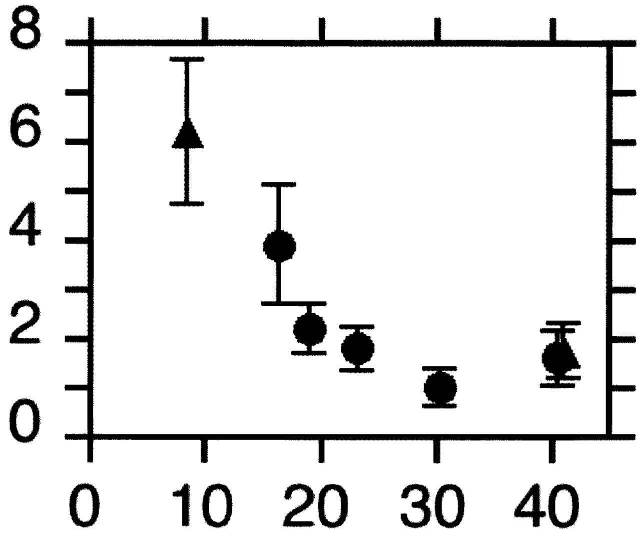


FIGURE 5C

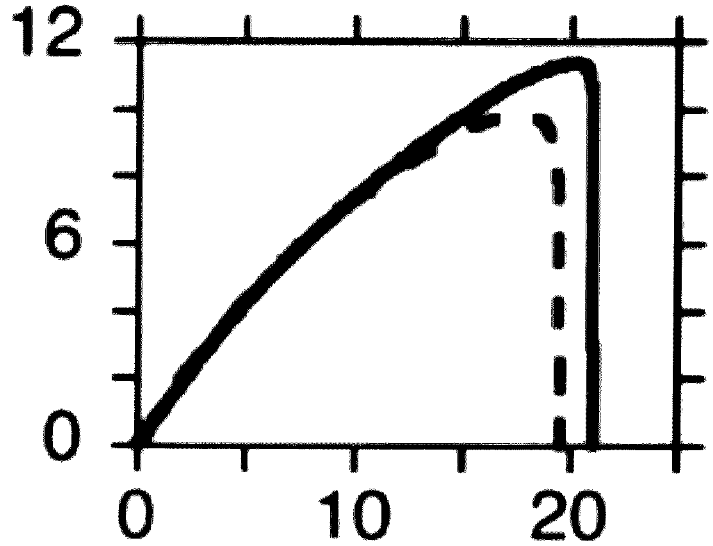


FIGURE 5D

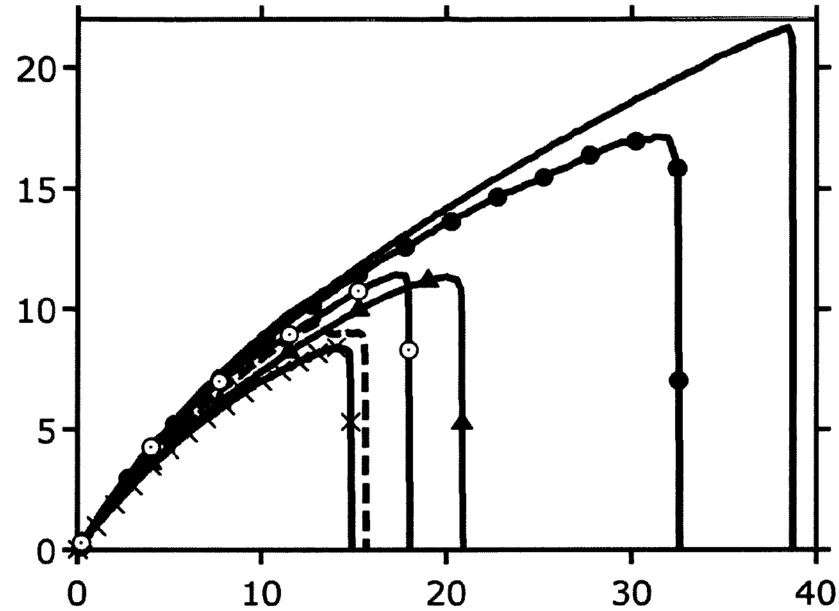


FIGURE 6

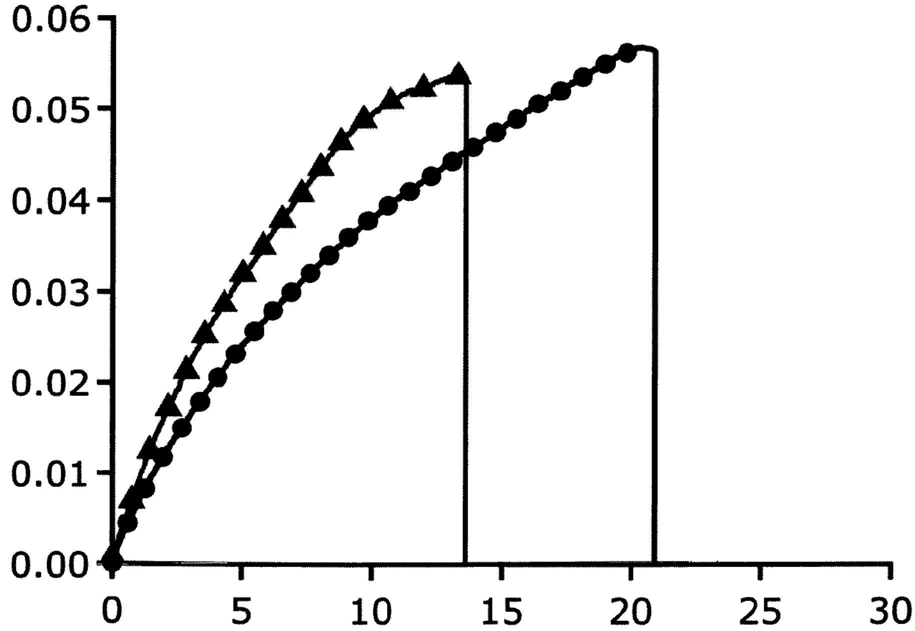


FIGURE 7A

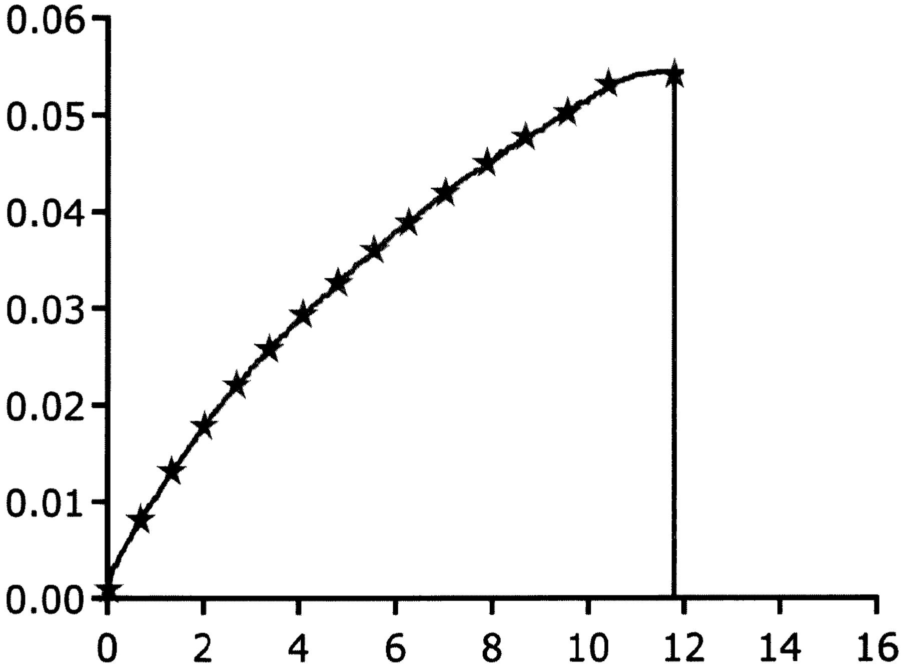


FIGURE 7B

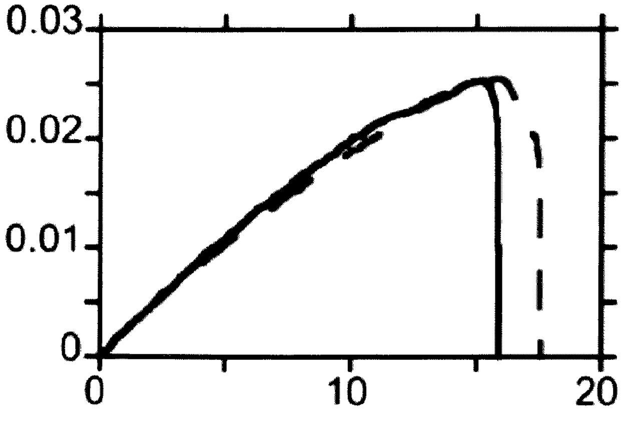


FIGURE 8

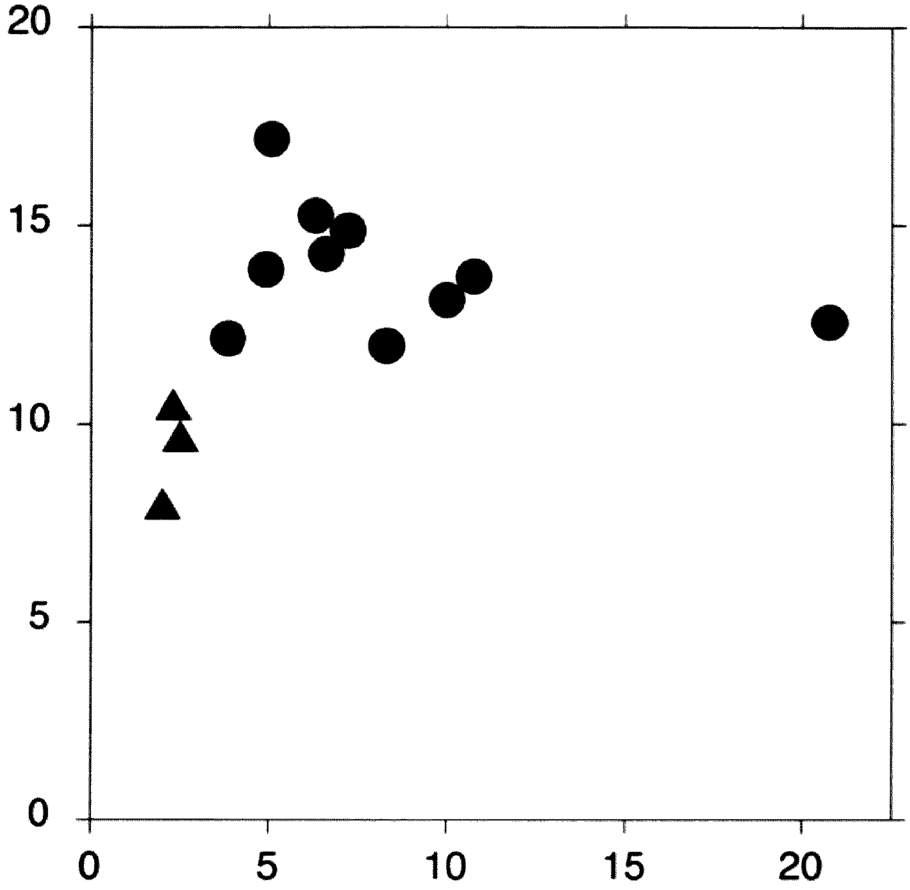


FIGURE 9A

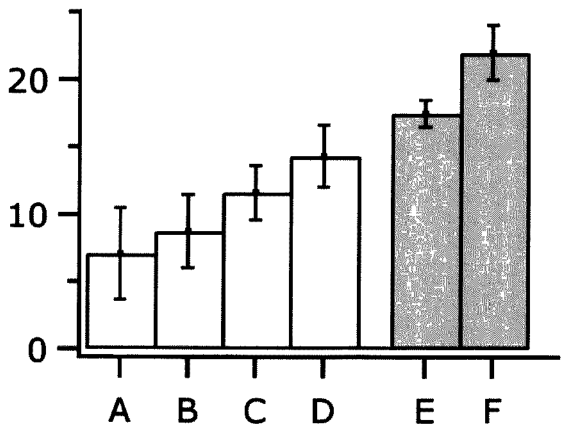


FIGURE 9B

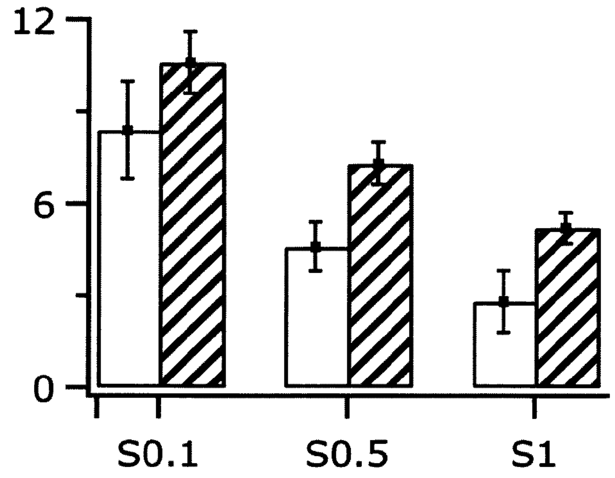


FIGURE 9C

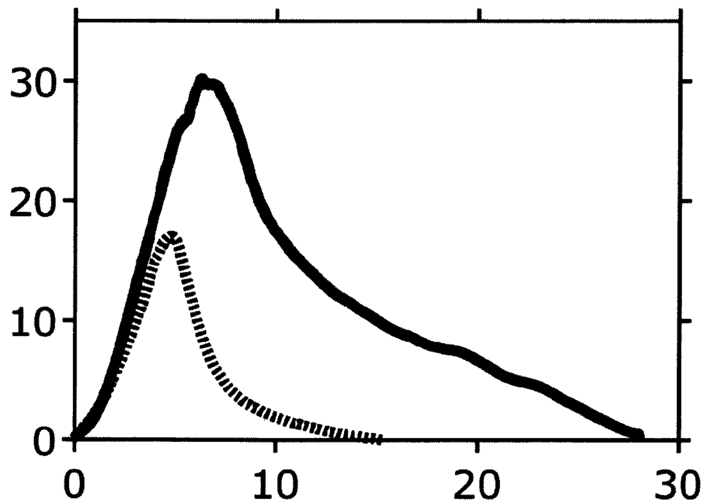


FIGURE 10

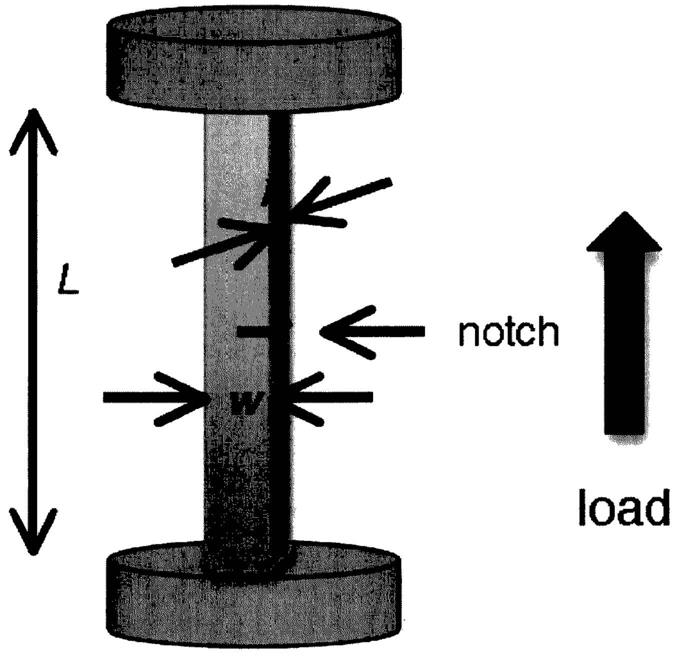


FIGURE 11

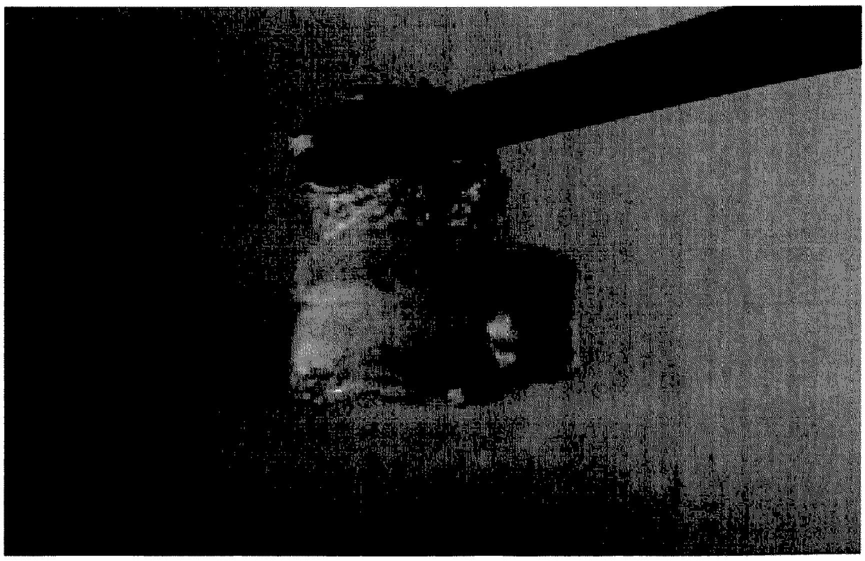

FIGURE 12

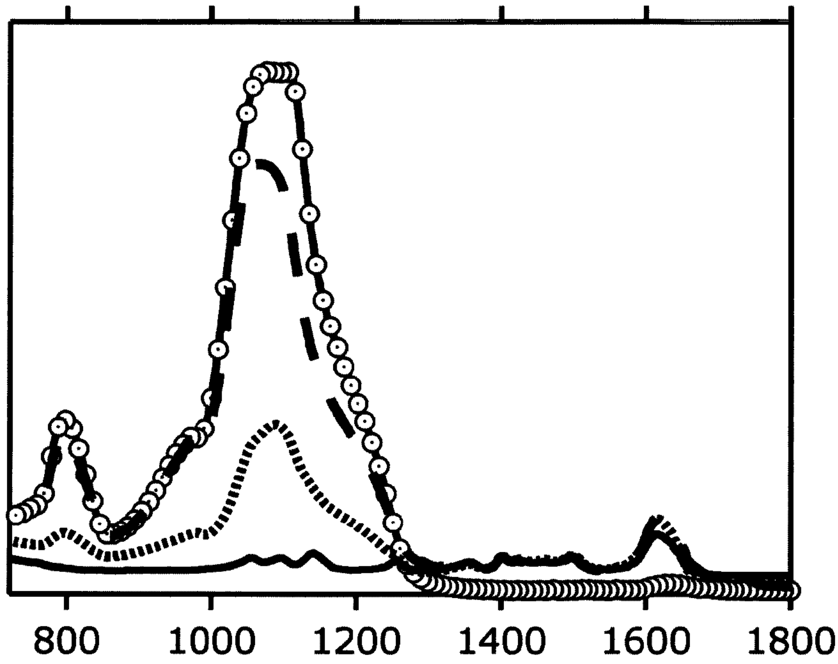


FIGURE 13

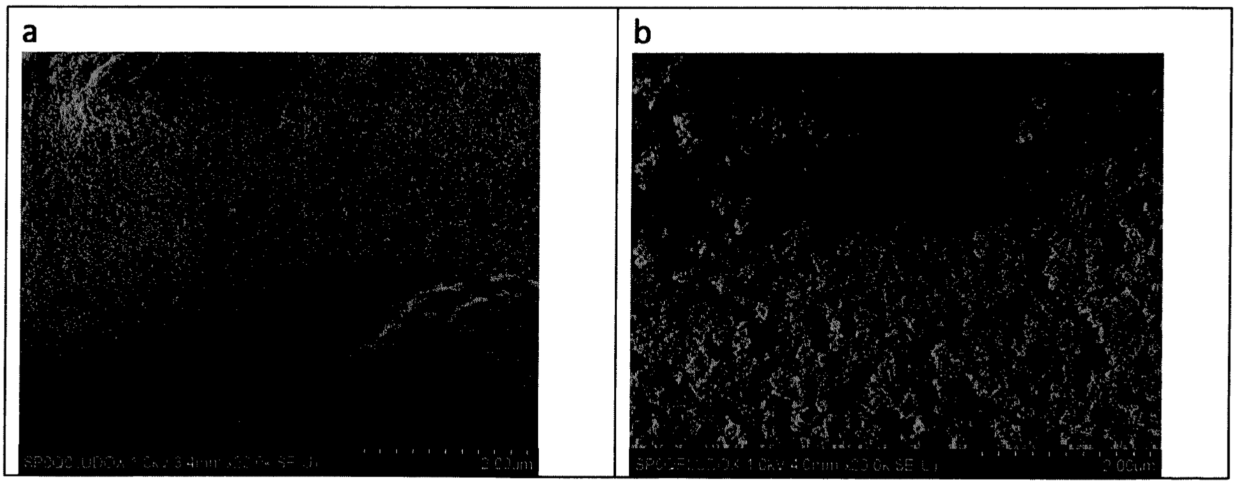


FIGURE 14

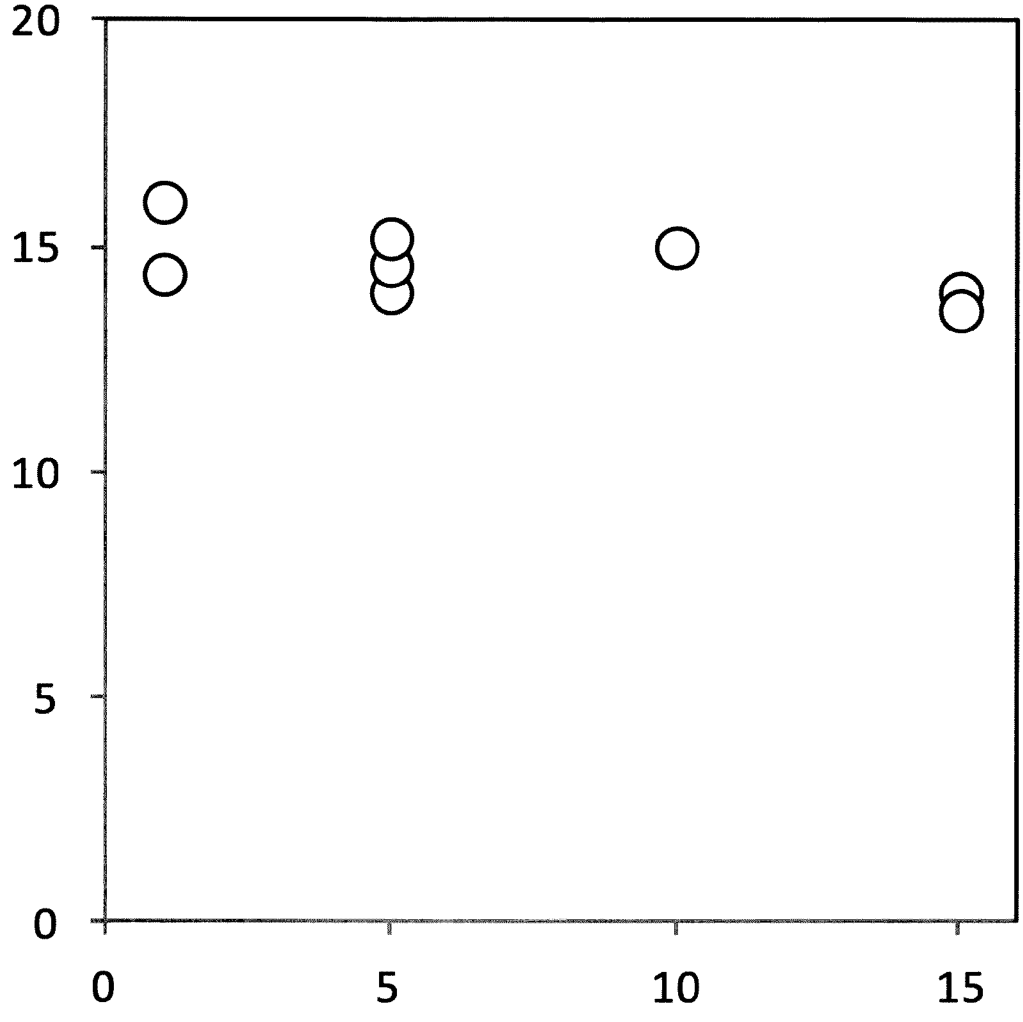


FIGURE 15

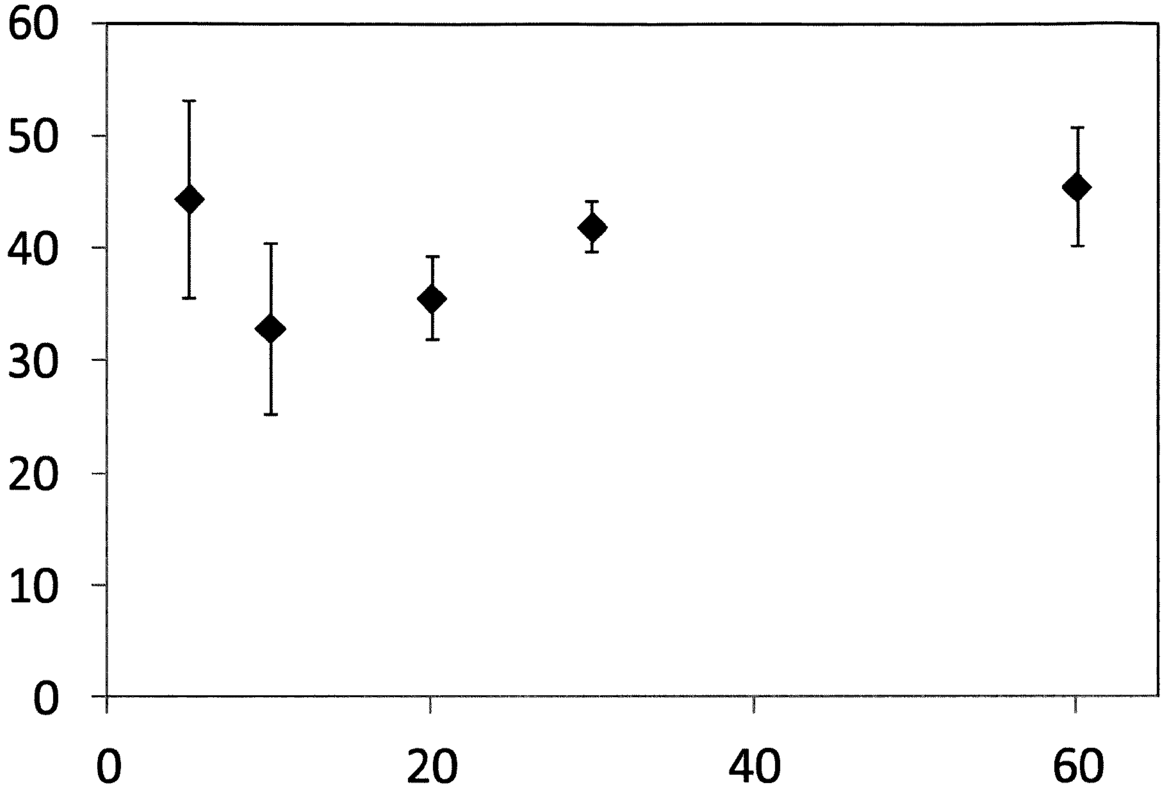


FIGURE 16

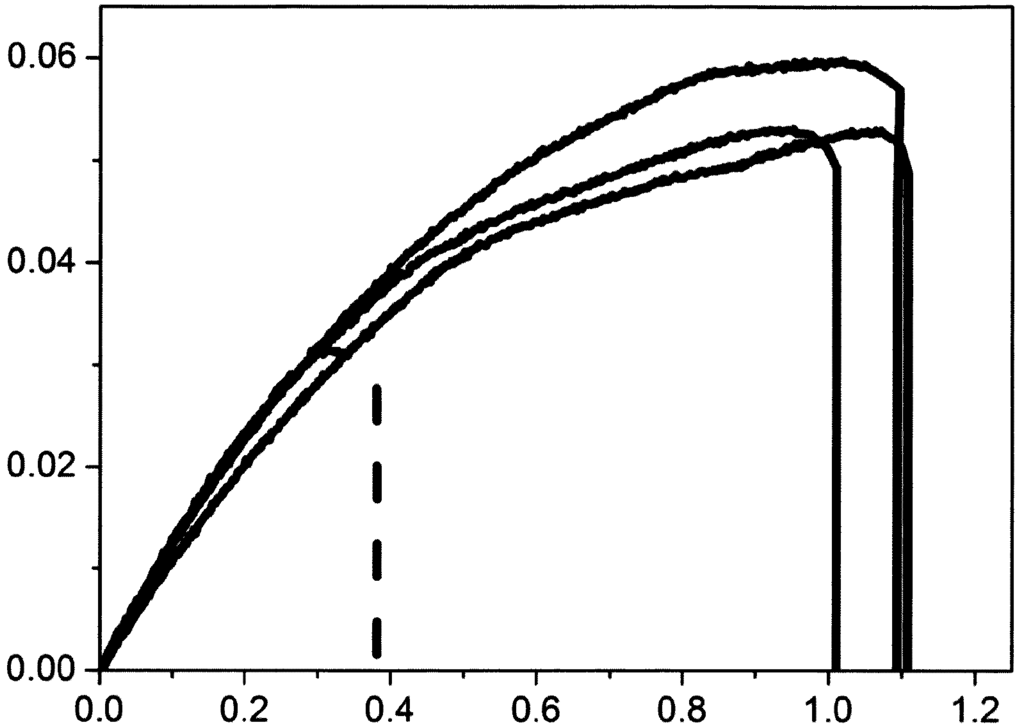


FIGURE 17

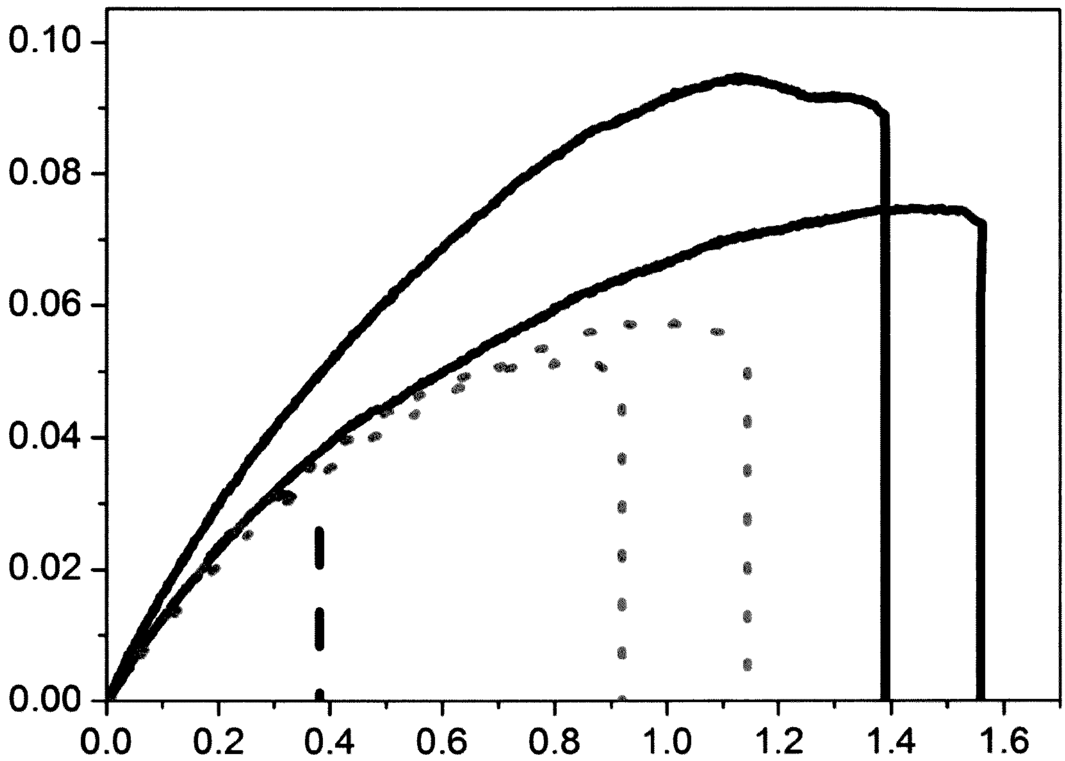


FIGURE 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004045494 A
- WO 2006012541 A
- WO 03026481 A
- US 2009275526 A
- US 20120220911 A


### Non-patent literature cited in the description


- **KENDALL, K.** Molecular Adhesion and Its Applications. Plenum Publishing Corporation, 2001
- **LAKE, G. J. ; THOMAS, A. G.** *Proceedings of the Royal Society A: Mathematical, Physical and Engineering Sciences,* 1967, vol. 300, 108-119
- **DE GENNES, P. G.** *Langmuir,* 1996, vol. 12, 4497-4500
- **SAHLIN, J. J. ; PEPPAS, N. A.** *Journal of Biomaterials Science, Polymer Edition,* 1997, vol. 8, 421-436
- **TAMAGAWA, H. ; TAKAHASHI, Y.** *Materials Chemistry and Physics,* 2008, vol. 107, 164-170
- **SAITO, J. et al.** *Polymer Chemistry,* 2011, vol. 2, 575
- **TECHAWANITCHAI, P.** *Soft Matter,* 2012, vol. 8, 2844
- **GONG, J. P.** *Soft Matter,* 2006, vol. 2, 544
- **PEZRON, E. ; RICARD, A. ; LEIBLER, L.** *J. Polym. Sci. B Polym. Phys.,* 1990, vol. 28, 2445-2461
- **BOSMAN, A. W. ; SIJBESMA, R. P. ; MEIJER, E. W.** *Materials Today,* 2004, 34-39
- **REUTENAUER, P. ; BUHLER, E. ; BOUL, P. J. ; CANDAU, S. J. ; LEHN, J.-M.** *Chemistry,* 2009, vol. 15, 1893-1900
- **NICOLAY, R. ; KAMADA, J. ; VAN WASSEN, A. ; MATYJASZEWSKI, K.** *Macromolecules,* 2010, vol. 43, 4355-4361
- **IMATO, K. et al.** *Angew. Chem. Int. Ed.,* 2011, vol. 51, 1138-1142
- **CORDIER, P. ; TOURNILHAC, F. ; SOULIÉ-ZIAKOVIC, C. ; LEIBLER, L.** *Nature,* 2008, vol. 451, 977-980
- **MAES, F. et al.** *Soft Matter,* 2012, vol. 8, 1681-1687
- **BAIT N. et al.** *Soft Matter,* 2011, vol. 7, 2025-2032
- **AGUZZI et al.** *Applied Clay Science,* 2007, vol. 36 (1-3), 22-36
- **WANG, Q. et al.** *Nature,* 2010, vol. 463, 339-343
- **HARAGUCHI, K. ; UYAMA, K. ; TANIMOTO, H.** *Macromol. Rapid Commun.,* 2011, vol. 32, 1253-1258
- **CARLSSON, L. ; ROSE, S. ; HOURDET, D. ; MARCELLAN, A.** *Soft Matter,* 2010, vol. 6, 3619-3631
- **GAHARWAR, A. K. ; RIVERA, C. P. ; WU, C.-J. ; SCHMIDT, G.** *Acta Biomaterialia,* 2011, vol. 7, 4139-4148
- **DUARTE A.P. et al.** *Progress in Polymer Science,* 2012, vol. 37, 1031-1050
- **JOHNSEN, B. B. ; A. J. KINLOCH et al.** Toughening mechanisms of nanoparticle-modified epoxy polymers. *Polymer,* 2007, vol. 48 (2), 530-541
- **KINLOCH, A. J.** Adhesion and Adhesives: Science and Technology. Springer, 1987
- **GENT, A. N. ; HAMED, G. R. ; HUNG, W. J.** Adhesion of elastomer layers to an interposed layer of filler particles. *The Journal of Adhesion,* 2003, vol. 79 (10), 905-913
- **LEE, B. P. ; MESSERSMITH, P. B. ; ISRAELACHVILI, J. N. ; WAITE, J. H.** Mussel-Inspired Adhesives and Coatings. *Annual Review of Materials Research,* 2011, vol. 41, 99-132
- **KINLOCH, A. J.** Adhesion and Adhesives: Science and Technology. Chapman and Hall, 1987
- **ORTS-GIL, G. ; K. NATTE et al.** *Journal of Nanoparticle Research,* 2011, vol. 13 (4), 1593-1604
- **ALEXANDRIDIS, P. ; B. LINDMAN.** Amphiphilic Block Copolymers: Self-Assembly and Applications. Elsevier Science, 2000
- **HUNTER, R. J. ; L. R. WHITE.** Foundations of colloid science. Clarendon Press, 1987
- **HOURDET, D. ; L. PETIT.** Macromolecular Symposia. Wiley-V C H Verlag Gmbh, 2010, vol. 291-292, 144-158
- **WISNIEWSKA, M.** *Journal of Thermal Analysis and Calorimetry,* 2010, vol. 101 (2), 753-760
- *Journal of colloid and interface science,* 1968, vol. 26, 62

- **W. STÖBER W. ; A. FINK ; E. BOHN.** Controlled growth of monodisperse silica spheres in the micron size range. *Journal of colloid and interface science,* 1968, vol. 26, 62-69
- **TOON T.-J. ; KIM J.S. ; KIM B.G. ; YU K.N. ; CHO M.-H. ; LEE J.-K.** Multifunctional nanoparticles possessing a "magnetic motor effect" for drug or gene delivery. *Angew. Chem. Int. Ed.,* 2005, vol. 44, 1068-1071
- **PREVOTEAU, A. ; SOULIÉ-ZIAKOVIC, C. ; LEIBLER, L.** Universally Dispersible Carbon Nanotubes. *J. Am. Chem. Soc.,* 2012, vol. 134, 19961-19964
- **BONDESON, D. ; MATHEW, A. ; OKSMAN, K.** Optimization of the isolation of nanocrystals from microcrystalline cellulose by acid hydrolysis. *Cellulose,* 2006, vol. 13, 171-180
- **CARLSSON, L. ; ROSE, S. ; HOURDET, D. ; MARCELLAN, A.** Nano-hybrid self-crosslinked PDMA/silica hydrogels. *Soft Matter,* 2010, vol. 6, 3619-3631
- **J. BASTIDE ; C.PICOT, S.** Candau The influence of pendent chains on the thermodynamic and viscoelastic properties of swollen networks. *J.Polym.Sci. :Physics Ed.,* 1979, vol. 17, 1441-1456
- **KENDALL, K.** Cracking of Short Lap Joints. *The Journal of Adhesion,* 1975, vol. 7, 137-140
- **RIVLIN, R.S.** Large Elastic Deformations of Isotropic Materials .4. Further Developments of the General Theory. *Philosophical Transactions of the Royal Society of London Series a-Mathematical and Physical Sciences,* 1948, vol. 241 (835), 379-397
- **GREENSMITH, H.W.** Rupture of rubber. X. The change in stored energy on making a small cut in a test piece held in simple extension. *Journal of Applied Polymer Science,* 1963, vol. 7 (3), 993-1002
- **PEFFERKORN, E. ; A. CARROY et al.** *Macromolecules,* 1985, vol. 18 (11), 2252-2258
- **WISNIEWSKA, M.** Temperature effect on adsorption properties of silica-polyacrylic acid interface. *Journal of Thermal Analysis and Calorimetry,* 2010, vol. 101 (2), 753-760
- **PARIDA SK ; DASH S ; PATEL S ; MISHRA BK.** *Advances in Colloid and Interface Science,* 2006, vol. 121 (1-3), 77-110
- **SEKINE Y ; IKEDA-FUKAZAWA T.** *Journal of Chemical Physics,* 2009, vol. 130, 3
- **HOURDET, D. ; PETIT, L.** *Macromol. Symp.,* 2010, vol. 291-292, 144-158
- **GRIOT, O. ; KITCHENER, J. A.** Role of surface silanol groups in the flocculation of silica suspensions by polyacrylamide. Part 1. Chemistry of the adsorption process. *Trans. Faraday Soc.,* 1965, vol. 61, 1026
- **JOHNER, A. ; JOANNY, J.-F.** Adsorption of polymeric brushes: Bridging. *J. Chem.Phys.,* 1992, vol. 96, 6257
- **P.G. DE GENNES.** Polymers at an interface; a simplified view. *Advances in Colloid and Interface Science,* 1987, vol. 27, 189-209
- **K. KENDALL.** *J. Phys. D: Applied Physics,* 1975, vol. 8, 512